# EUROPEAN PATENT APPLICATION

(11) **EP 1 757 289 A1**
(43) Date of publication of application: **28.02.2007**
(21) Application number: 05734453.3
(22) Date of filing: 19.04.2005
(51) Int. Cl.: A61K 31/405

(54) **COMPOSITION FOR ACCELERATION OF ALCOHOL METABOLISM OR RECUPERATION FROM FATIGUE THROUGH GLUCONEOGENESIS**

(30) Priority: 19.04.2004 JP 2004123427
(71) Applicant: KIRIN BEER KABUSHIKI KAISHA, Tokyo 104-8288 (JP)
(72) Inventor: KATAYAMA, Yumiko, Kirin Beer Kabushiki Kaisha, Kanazawa-ku Yokohama-shi Kangawa 2360004 (JP); YOSHIDA, Aruto, Kirin Beer Kabushiki Kaisha, Kanazawa-ku Yokohama-shi Kangawa 2360004 (JP)
(74) Representative: HOFFMANN EITLE
(86) International application number: PCT/JP2005/007475
(87) International publication number: WO 2005/102321

(57) **Abstract**

There is provided a composition useful for mitigating a physical burden and recovering from fatigue at the time of alcohol intake, within a safety dose of active ingredients. The composition is for accelerating alcohol metabolism and for relieving fatigue by gluconeogenesis *in vivo,* and is **characterized by** comprising an effective amount of caffeine and an effective amount of an amino acid(s) or a peptide(s) selected from the group consisting of serine, tryptophan, serine rich peptides, tryptophan rich peptides, serine/tryptophan rich peptides and mixtures thereof, as active ingredients.

SEQUENCE LISTING

<110> Kirin Beer Kabushiki Kaisha

<120> Composition for accelerating alcohol
      metabolism or relieving fatigue by
      gluconeogenesis

<130> PH-2451-PCT

<140>
<141>

<150> JP 2004-123427
<151> 2004-04-19

<160> 8

<170> PatentIn Ver. 2.1

<210> 1
<211> 24
<212> DNA
<213> Artificial Sequence

<220>
<223> Description of Artificial Sequence:sense primer
      for SDS

<400> 1

acgctgccgt caaggaagga aagc 24

<210> 2
<211> 25
<212> DNA
<213> Artificial Sequence

<220>
<223> Description of Artificial Sequence:antisense
      primer for SDS

<400> 2
gcgggctcca ccaggatctt ctcat 25

<210> 3
<211> 24
<212> DNA
<213> Artificial Sequence

<220>
<223> Description of Artificial Sequence:sense primer
      for TDO

<400> 3
gctgctgaaa tcggagcagg agca 24

<210> 4

<211> 24

<212> DNA

<213> Artificial Sequence

<220>

<223> Description of Artificial Sequence:antisense

primer for TDO

<400> 4

ctgcttccgg aactctgcca tctg 24

<210> 5

<211> 22

<212> DNA

<213> Artificial Sequence

<220>

<223> Description of Artificial Sequence:sense primer

for ALT1

<400> 5

tcgaggccgt aatccgcttt gc 22

<210> 6

<211> 22

<212> DNA

<213> Artificial Sequence

<220>
<223> Description of Artificial Sequence:antisense
      primer for ALT1

<400> 6
gaaacccgca ctcgcccatg ta                                              22

<210> 7
<211> 23
<212> DNA
<213> Artificial Sequence

<220>
<223> Description of Artificial Sequence:sense primer
      for PO

<400> 7
ggggaacgtg ggctttgtgt tca                                             23

<210> 8
<211> 24
<212> DNA
<213> Artificial Sequence

<220>
<223> Description of Artificial Sequence:antisense
      primer for PO

<400> 8

tggtgatgcc caaagcttgg aaga 24

## Description

### Technical Field

The present invention relates to a composition for accelerating alcohol metabolism or relieving fatigue by gluconeogenesis, comprising caffeine and serine, tryptophan, or a serine and/or tryptophan-rich peptide(s), as active ingredients. The composition of the present invention is particularly useful in accelerating alcohol metabolism and/or relieving fatigue.

### Background Art

Alcoholic drinks such as alcohol beverages are effective in relaxing person's mind, thereby improving communications. Therefore they have been long used in a wide variety of occasions such as religious serves, ceremonies, and parties, etc. Furthermore, the alcohol beverages have not only good tastes in itself but also effective roles in improving taste of meals, releasing stress and maintaining health, so that many peoples are fond of them in their daily lives.

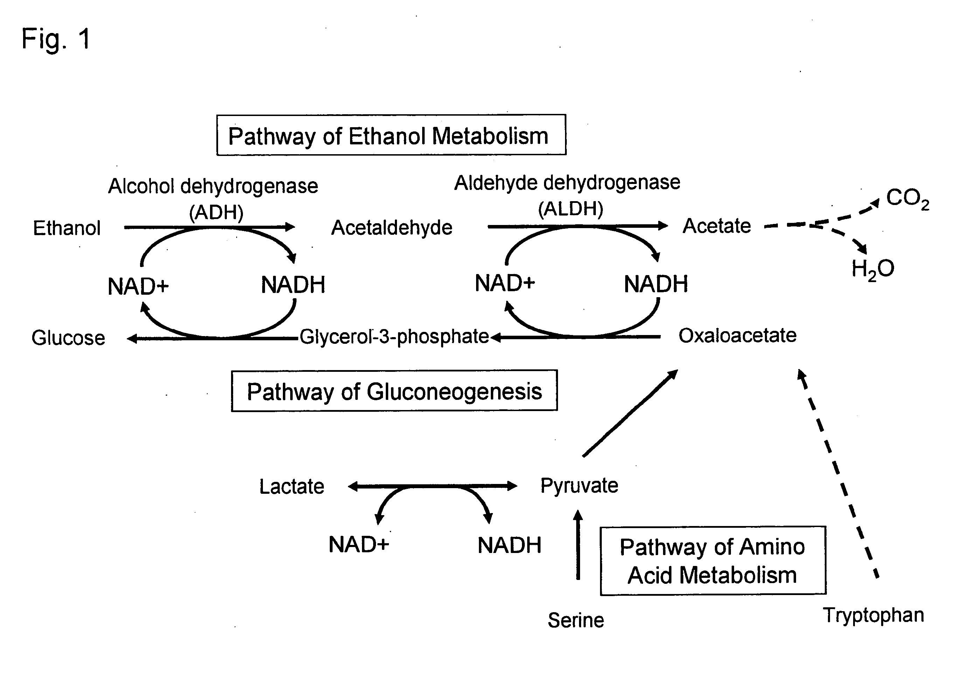

Ethanol contained in an alcohol beverage is metabolized mainly in the liver with alcohol dehydrogenase (ALD) into acetaldehyde, which is further metabolized into acetate by aldehyde dehydrogenase (ALDH). These two enzymes (ADH and ALDH) require oxidized form nicotinamide-adenine dinucleotide (hereinafter referred to as "NAD⁺") in each of the reaction process of the enzymes. The NAD⁺ used in the reaction is reduced to a reduced form of nicotinamide-adenine dinucleotide (hereinafter referred to as "NADH") (Fig. 1). Therefore, when ethanol is excessively taken, the ratio of NADH/NAD⁺ increases as ethanol is metabolized. As a result, the intracellular environment leans toward a reduction state, suppressing metabolization of ethanol from proceeding. To recover from the over-reduction state, the reaction from pyruvate to lactate, which is accompanied by the oxidation of NADH to NAD⁺, proceeds within the cells. Consequently, the *in vivo* ratio of lactate to pyruvate increases and a burden is imposed on the body. Particularly, when an extraordinary excessive amount of ethanol is taken, a blood lactate level greatly increases, inducing hyperlactacidemia, which is known to be at a high risk of causing acidosis (academia) and hyperuricemia. Furthermore, when pyruvate decreases, gluconeogenesis is prevented, with the result that not only alcoholic hypoglycemia takes place but also the circulation of the tricarboxylic acid cycle for producing energy degrades.

It is known that the ratio of light drinkers is high in Mongoloids including Japanese as compared to Westerners. This is because the ratio of the persons having incomplete ALDH2 function is high, which is one of two types of ALDHs and has higher affinity for acetaldehyde. A person called a "light drinker" has a genotype of ALDH2*1/2 (ND) (one of ALDH2 alleles loses its function) and occupies about 30 to 40% of the entire population. A person "intolerance for alcohol" has a genotype of ALDH2*1/1 (DD) (both of the ALDH2 allelic genes lose their functions) and occupies about 10% of the entire population. The reason why persons of the ND and DD genotypes have a low tolerance for alcohol is that although ethanol taken from an alcohol beverage can be metabolized up to acetaldehyde, acetaldehyde, which is toxic and causes uncomfortable feeling such as "drunken sickness" and "hangover", is not easily metabolized *in vivo.*

From the background mentioned above, it has been strongly desired over years to develop a useful composition that allows a person to comfortably get drunk without having a large physical burden and appropriately get sober, when an alcohol beverage is taken. To satisfy the desire, many researchers have developed ethanol absorption inhibitors and ethanol metabolism accelerators. For example, as shown below, the findings on amino acids and peptides having an effect upon alcohol metabolism have been hitherto disclosed. Examples of amino acids and peptides accelerating alcohol metabolism include glycine (Japanese Patent Publication (Kokai) No. 2003-116504), D-cysteine and/or a salt thereof (Japanese Patent Publication (Kokai) No. 3-74327), lysine (Japanese Patent Publication (Kokai) No. 6-116144), a peptide having a molecular weight of 200 to 4000 and obtained by enzymatic decomposition of corn protein (Japanese Patent Publication (Kokai) No. 7-285881), and a processed pork product obtained by treating pork meat with protease (Japanese Patent Publication (Kokai) No. 11-276116). Examples of amino acids and peptides suppressing the toxicity of acetaldehyde include L-cysteine (Sprince H et al., Agents Actions (1975) vol. 5 164-173), L-alanine (Japanese Patent Publication (Kokai) No. 61-134313), dipeptide containing L-alanine (Japanese Patent Publication (Kokai) No. 4-21635) and L-theanine (Japanese Patent Publication (Kokai) No. 6-40901). Furthermore, examples of the amino acids and peptides inhibiting generation of acetaldehyde include proline (Japanese Patent Publication (Kokai) No. 6-116144). Moreover, examples of the amino acids and peptides having an anti-alcoholism effect include alanine and glutamine (Japanese Patent Publication (Kokoku) No. 4-20409). However, the effects of these amino acids and peptides are mostly confirmed based on the results of administrations at large amounts to animals as well as based on human studies not satisfying scientific, quantitative or statistic requirements. There are still few amino acids and peptides whose effects were confirmed in a dose which a human can easily take in one's daily life.

On the other hand, it is also disclosed that a component containing catechin and/or caffeine extracted from tea as a main ingredient has an effect of accelerating ethanol metabolism (Japanese Patent Publication (Kokai) Nos. 6-263648 and 8-169831). From studies of administration to mice, it is demonstrated that the active ingredient extracted from tea, which is primarily caffeine, must be administered in an amount as relatively large as 300 mg/kg body weight in order to significantly reduce the blood ethanol level (Kakuda T et al. Biosci. Biotech. Biochem. (1996) vol 60 1450-1454). Studies further conducted thereafter show that the ethanol absorption suppression action in the stomach greatly contributes to reduction of the blood ethanol level observed when caffeine is administered to mice in an amount of 300 mg/kg body weight (Japanese Patent Publication (Kokai) No. 8-277221). It is an interest finding that a blood ethanol level is reduced by caffeine. However, the pharmaceutically acceptable dose of caffeine greatly differed from the dose providing a significant change in mice. To explain more specifically, a desirable amount of caffeine to be taken by a human adult is about 100 to 300 mg/dose, and the total amount of caffeine per day is desirably 500 mg or less. The safety dose of caffeine to be administered to an adult female having an average weight of about 50 kg is computationally obtained as 2 to 6 mg/kg body weight per day. In addition, the mechanism how caffeine reduces a blood ethanol level has not yet been fully elucidated. It is therefore not always easy to calculate an effective amount of caffeine in humans based on the experimental results in mice. A question as to whether caffeine at a safety dose is really effective still remains unsolved.

Serine is known to have a skin moisturizing effect and an anti-aging effect of the brain. Tryptophan is known to serve as a precursor of a neurotransmitter within the brain, so that it has depression relieving action and ataractic, hypnotic, sedative and analgesic effects. Naturally occurring amino acids except leucine and lysine are known as amino acids involved in gluconeogenesis (i.e., amino acids convertible into glucose). However, it has not yet been known that serine and tryptophan work in concert with caffeine to selectively serve as effective substrates in gluconeogenesis, enhancing acceleration of ethanol metabolism.

### Disclosure of the Invention

When Mongoloids including Japanese are compared to Westerners, the ratio of light drinkers is high. Therefore, it has been strongly desired over years to develop a useful composition that allows a person to comfortably get drunk without having a large physical burden when an alcohol beverage is taken. It is a not rare case even for a hard drinker to have a physical burden and uncomfortable feeling depending upon the physical conditions and the amount of an alcohol beverage. However, almost all the compositions hitherto disclosed have problems. Some of them must take a large amount and others are questionable in the practical effects in humans.

The present invention has been made in view of the aforementioned circumstances and directed to provide a composition useful for accelerating alcohol metabolism, mitigating a physical burden when alcohol (i.e., ethanol) is taken, or recovering from fatigue, with a safety dose of an active ingredient.

We investigated the ethanol metabolism accelerating effect of caffeine when it is taken in a dose of 3 mg/kg body weight, which falls within the acceptable intake range of a human. However, a significant effect was not observed. Then, we investigated the effect of caffeine intake on the liver, which is a main organ involved in ethanol metabolism, by use of rats. As a result, we found that catabolism of specific amino acids such as serine and tryptophan is accelerated based on change of gene expression by caffeine administration. We actually measured a blood glucose level. Based on the measurement, it was estimated that gluconeogenesis and the metabolic system (Fig. 1) from an amino acid to glucose were accelerated. Furthermore, possibility was demonstrated that a series of the metabolic system, since it is accompanied by the conversion of NADH to NAD⁺, suppresses an increase in the ratio of NADH/NAD⁺ at the time of ethanol intake, smoothly metabolizing ethanol. Based on these findings, they conceived an idea that an ethanol metabolism acceleration effect may be remarkably produced by combining caffeine and an amino acid (serine or tryptophan), which exhibit no apparent ethanol metabolism acceleration effect within a safety dose for a human, and demonstrated the idea by a human study.

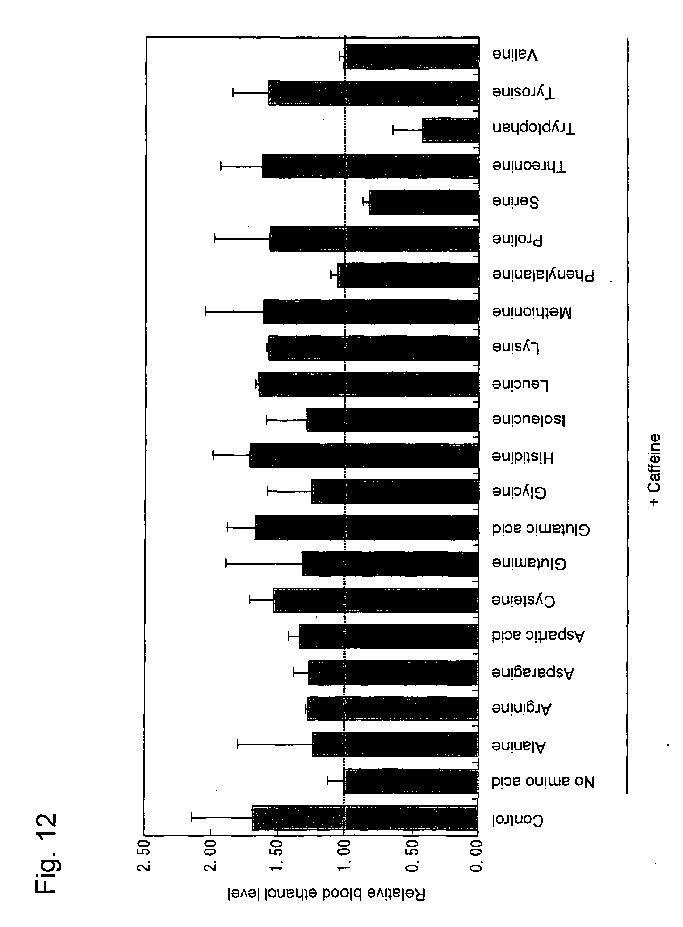

As a result, the human study showed that an apparent ethanol metabolism acceleration effect is not observed even if a safety dose of caffeine or amino acid (serine) is separately taken. However, a significant reducing effect of a blood ethanol level was observed for the first time by taking caffeine and the amino acid at the same time. In addition, a down tendency of a blood acetaldehyde level was observed. From this, it was concluded that a blood ethanol level was reduced because the metabolism of ethanol into acetate was accelerated. Furthermore, the glucose level in blood significantly increased and the ratio of NADH/NAD⁺ has a downward tendency. From these facts, it was deduced that acceleration of gluconeogenesis was involved in accelerating the ethanol metabolism. In the subjective evaluation concomitantly performed, the number of persons who had "comfortable feeling" at the time of ethanol intake significantly increased. Taking all results into consideration, a composition comprising a safety dose of caffeine and an effective amount of serine or tryptophan in combination is extremely advantageous since it realizes augmentation of feeling of "comfortable feeling" and mitigation of a physical burden at the time of alcohol intake, at a one stroke. Furthermore, it is considered that the ethanol-metabolism accelerating effect results in preventing "hangover" and that the acetaldehyde reduction effect results in preventing "drunken sickness". Note that intensive investigation was conducted to find the type of amino acid that produces an effect in combination with caffeine. As a result, serine or tryptophan was prominently effective (Fig. 12).

Based on the aforementioned findings, we have now found that mitigation of a physical burden at the time of alcohol intake and improvement from hypoglycemia at the time of fatigue can be attained by combining caffeine and an amino acid (serine or tryptophan), which exhibit no apparent ethanol metabolism acceleration effect within a safety dose for a human (i.e., acceptable range).

Accordingly, the present invention comprises the followings.

According to a first aspect of the invention, there is provided a composition for cooperatively accelerating alcohol metabolism and gluconeogenesis *in vivo,* characterized by comprising an effective amount of caffeine and an effective amount of an amino acid(s) and/or a peptide(s) selected from the group consisting of serine, tryptophan, serine rich peptides, tryptophan rich peptides, serine/tryptophan rich peptides and mixtures thereof, as active ingredients.

In an embodiment, the composition of the present invention is used for mitigating a physical burden arising due to alcohol intake and/or accelerating alcohol metabolism.

In another embodiment, the composition of the present invention contains caffeine and serine as active ingredients.

In another embodiment, the composition of the present invention contains caffeine and tryptophan as active ingredients.

In another embodiment, the composition of the present invention contains caffeine and a mixture of serine and tryptophan as active ingredients.

In another embodiment, the serine rich peptide contains a serine residue in an amount of 50 to 100% of the amino acids constituting the peptide.

In another embodiment, the serine-rich peptide is seryl-serine.

In another embodiment, the tryptophan rich peptide contains a tryptophan residue in an amount of 50 to 100% of the amino acids constituting the peptide.

In another embodiment, the tryptophan-rich peptide is tryptophanyl-tryptophan.

In another embodiment, the total number of serine residues and tryptophan residues of the serine/tryptophan rich peptide corresponds to 50% to 100% of the amino acids constituting the peptide.

In another embodiment, the serine/tryptophan-rich peptide is seryl-tryptophan or tryptophanyl-serine.

In another embodiment, the content of caffeine is 10 mg or more.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg or more per composition.

In another embodiment, the content of caffeine falls within an acceptable intake range of a human per composition.

In another embodiment, the content of caffeine is 500 mg or less per composition.

In another embodiment, the content of caffeine is 50 mg to 500 mg per composition.

In another embodiment, the content of the amino acid is 100 mg or more per composition.

In another embodiment, the content of the amino acid is 100 mg to 20 g per composition.

In another embodiment, the content of the peptide is 100 mg or more per composition.

In another embodiment, the content of serine of the composition of the present invention is 500 mg or more per composition in terms of free serine.

In another embodiment, the content of serine of the composition of the present invention is 1 g or more per composition in terms of free serine.

In another embodiment, the composition of the present invention is a beverage or food or medicament.

In another embodiment, the beverage or food is an amino acid beverage containing caffeine and serine as described above.

According to a second aspect of the present invention, there is further provided a composition containing 500 mg or less of caffeine and 100 mg or more of a combination consisting of at least 2 components selected from the group consisting of serine or serine rich peptides, tryptophan or tryptophan rich peptides and, and serine/tryptophan rich peptides.

In an embodiment, the content of serine in the combination is 100 mg or more per composition in terms of free serine.

In another embodiment, the content of serine in the combination is 500 mg or more per composition in terms of free serine

In another embodiment, the content of serine in the combination is 1 g or more per composition in terms of free serine.

In another embodiment, the composition of the present invention is a beverage or food or medicament.

In another embodiment, the content of caffeine is 10 to 500 mg.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg to 500 mg (both inclusive) per composition.

In another embodiment, all embodiments regarding the contents and types of the active ingredients according to the first aspect are applied to the present invention.

According to a third aspect of the present invention, there is provided a composition containing 500 mg or less of caffeine and 100 mg or more of a serine/tryptophan rich peptide.

In an aspect, the composition of the present invention is a beverage or food or medicament.

In another embodiment, the content of caffeine is 10 to 500 mg.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg to 500 mg (both inclusive) per composition.

In another embodiment, all embodiments regarding the contents and types of the active ingredients according to the first aspect are applied to the present invention.

According to a fourth aspect of the present invention, there is provided a composition for relieving fatigue, characterized by comprising an effective amount of caffeine and an effective amount of an amino acid(s) and/or a peptide(s) selected from the group consisting of serine, tryptophan, serine rich peptides, tryptophan rich peptides, serine/tryptophan rich peptides and mixtures thereof, as active ingredients.

In an embodiment, the composition of the present invention is used for mitigating a physical burden arising due to alcohol intake.

In another embodiment, the composition of the present invention is used for relieving fatigue arising due to hypoglycemia.

In another embodiment, the composition of the present invention contains caffeine and serine as active ingredients.

In another embodiment, the composition of the present invention is a beverage or food or a medicament.

. In another embodiment, the content of caffeine is 10 mg or more.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg or more per composition.

In another embodiment, all embodiments regarding the contents and types of the active ingredients according to the first aspect are applied to the present invention.

According to a fifth embodiment of the present invention, there is provided a composition for accelerating alcohol metabolism, characterized by comprising an effective amount of caffeine and an effective amount of an amino acid(s) and/or a peptide(s) selected from the group consisting of serine, tryptophan, serine rich peptides, tryptophan rich peptides, serine/tryptophan rich peptides and mixtures thereof, as active ingredients.

In an embodiment, the composition of the present invention comprises caffeine and serine, tryptophan or a mixture thereof as active ingredients.

In another embodiment, the content of serine of the composition of the present invention is 500 mg or more per composition in terms of free serine.

In another embodiment, the content of serine of the composition of the present invention is 1 g or more per composition in terms of free serine.

In another embodiment, the composition of the present invention is a beverage or food or a medicament.

In another embodiment, the content of caffeine is 10 mg or more.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg or more per composition.

In another embodiment, all embodiments regarding the contents and types of the active ingredients according to the first aspect are applied to the present invention.

According to a sixth aspect of the present invention, there is provided a composition set containing a first composition comprising 500 mg or less of caffeine and a second composition containing 100 mg or more of serine, tryptophan, or a mixture thereof in combination as discrete packages.

In an embodiment, the content of serine of the second composition is 500 mg or more.

In another embodiment, the content of serine in the second composition is 1 g or more.

In another embodiment, the composition of the present invention is beverage or food or medicament.

In another embodiment, the content of caffeine is 10 to 500 mg.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg to 500 mg (both inclusive) per composition.

In another embodiment, all embodiments regarding the contents and types of the active ingredients according to the first aspect are applied to the present invention.

According to a seventh aspect of the present invention; there is provided a caffeine containing beverage, characterized by comprising an amino acid(s) and/or a peptide(s) selected from the group consisting of serine, tryptophan, serine rich peptides, tryptophan rich peptides, serine/tryptophan rich peptides and mixtures thereof in an effective amount of 500 mg or more per beverage in terms of free serine and/or free tryptophan.

In an embodiment, the beverage is selected from the group consisting of green tea, black tea, coffee, cola and a drinkable preparation.

In another embodiment, the content of caffeine is 10 mg or more.

More specifically, examples of the content of caffeine include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg or more per beverage.

In another embodiment, the content of caffeine is 10 mg to 500 mg, both inclusive.

In another embodiment, the content of caffeine is 50 mg to 500 mg, both inclusive.

In another embodiment, the content of caffeine is 100 mg to 500 mg, both inclusive.

In another embodiment, the content of caffeine is 150 mg to 500 mg, both inclusive.

In another embodiment, the serine rich peptide and the tryptophan rich peptide respectively contain serine residues and tryptophan residues in an amount of 50% to 100% of the amino acids constituting the peptide.

In another embodiment, the total number of serine residues and tryptophan residues of the serine/tryptophan rich peptide corresponds to 50% to 100% of the amino acids constituting the peptide.

In another embodiment, the content of the amino acid and/or the peptide in the composition of the present invention is 1 g or more per composition in terms of free serine and/or free tryptophan.

In another embodiment, the content of the amino acid or the peptide or the mixture thereof in the composition of the present invention is 500 mg to 20 g per composition in terms of free serine and/or free tryptophan.

According to an eighth aspect of the present invention, there is further provided a composition for use in accelerating alcohol metabolism or relieving fatigue in mixing a caffeine containing beverage, characterized by adding an amino acid(s) and/or a peptide(s) selected from the group consisting of serine, tryptophan, serine rich peptides, tryptophan rich peptides, serine/tryptophan rich peptides and mixtures thereof in an amount of 100 mg or more, preferably 500 mg or more per dose in terms of free serine and/or free tryptophan (as supplements).

In an embodiment, the composition is sugar, milk or lemon juice containing serine and/or serine rich peptide in the amount mentioned above.

In another embodiment, the serine rich peptide and the tryptophan rich peptide respectively contain serine residues and tryptophan residues in an amount of 50% to 100% of the amino acids constituting the peptide.

In another embodiment, the total number of serine residues and tryptophan residues of the serine/tryptophan rich peptide corresponds to 50% to 100% of the amino acids constituting the peptide.

In another embodiment, the content of the amino acid and/or the peptide in the composition of the present invention is 1 g or more per composition in terms of free serine and/or free tryptophan.

In another embodiment, the content of the amino acid or the peptide or the mixture thereof in the composition of the present invention is 500 mg to 20 g per composition in terms of free serine and/or free tryptophan.

In another embodiment, the caffeine containing beverage contains caffeine in an amount of about 10 mg or more.

More specifically, examples of the content of caffeine in the caffeine containing beverage include, but are not limited to, 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, or 450 mg or more per composition.

### Advantages of the Invention

The composition of the present invention contains caffeine in an amount within an acceptable intake range of a human in combination with an effective amount of serine, tryptophan and serine/tryptophan rich peptide in combination. By virtue of this, the composition produces advantageous effects of accelerating the reaction from ethanol to acetate at the time of alcohol intake without stopping; at the same time, accelerating gluconeogenesis, thereby promoting alcohol metabolism, and mitigating a physical burden (e.g., uncomfortable feeling, collapse, etc.) at the time of alcohol intake, as well as augmenting "comfortable drunkenness" or "comfortable feeling". These advantageous effects are due to contribution of NADH to NAD⁺ conversion taking place in the gluconeogenesis metabolism system to acceleration of the ethanol metabolism.

The composition of the present invention accelerates gluconeogenesis, so that it effectively works in recovering from hypoglycemia and relieving fatigue.

The specification incorporates the contents described in the specification and/or drawings of Japanese Patent Application No. 2004-123427, based on which the priority of the present application is claimed.

### Brief Description of the Drawings

Fig. 1 shows a metabolic relationship in which the alcohol metabolism and gluconeogenesis works in concert with each other;
Fig. 2A shows an increase of the blood glucose level after caffeine administration in rats;
Fig. 2B shows a change of gene expression by caffeine, where TDO represents a tryptophan 2,3-dioxygeanse gene, SDS a serine dehydratase gene, and ALT an alanine-amino transferase gene (data are shown by mean±SD);
Fig. 3 shows a decrease of a blood ethanol level by caffeine + serine administration in rats (data are shown by mean±SE);
Fig. 4 shows the effect of serine (A), caffeine (B) and caffeine + serine (C) on a blood ethanol level in a human study (data are shown by mean±SE);
Fig. 5 shows the effect of serine (A), caffeine (B) and caffeine + serine (C) on a blood acetaldehyde level in a human study (data are shown by mean±SE);
Fig. 6 shows the effect of serine (A), caffeine (B) and caffeine + serine (C) on a blood acetate level in a human study (data are shown by mean±SE);
Fig. 7 shows a reduction of a blood lactate/pyruvate ratio by administration of caffeine + serine in a human study (data are shown by mean±SE);
Fig. 8 shows the effect of serine (A), caffeine (B) and caffeine + serine (C) on a blood glucose level in a human study (data are shown by mean±SE);
Fig. 9 shows the results of subjective evaluation on drunkenness after alcohol drinking by administration of caffeine + serine in a human study (data are shown by mean±SE);
Fig. 10 shows the results of subjective evaluation on comfortable and uncomfortable feeling after alcohol drinking by administration of caffeine + serine in a human study (data are shown by mean±SE);
Fig. 11 shows a reduction of a blood ethanol level by administration of caffeine + tryptophan in rats (data are shown by mean±SE);
Fig. 12 shows a change of a blood ethanol level by administration of caffeine and each of various types of amino acids in rats (data are shown by mean±SD);
Fig. 13 shows an ethanol metabolism acceleration effect of caffeine + serine in human (NN) study, in which Fig. 13A shows a change of a blood ethanol level with time; Fig. 13B shows a change of a blood acetaldehyde level with time; Fig. 13C shows a change of a blood acetate level with time; and Fig. 13D shows a change of a lactate/pyruvate ratio with time (data are shown by mean±SE);
Fig. 14 shows a relative blood ethanol level when caffeine + serine is orally administered (data are shown by mean±SD);
Fig. 15 shows an ethanol metabolism acceleration effect of caffeine + serine which is administered after ethanol intake in human (ND), in which Fig. 15A shows a change of a blood ethanol level with time; Fig. 15B shows a change of a blood acetaldehyde level with time; Fig. 15C shows a change of a blood acetate level with time; and Fig. 15D shows a change of a lactate/pyruvate ratio with time (data are shown by mean±SE);
Fig. 16 shows an ethanol metabolism acceleration effect of caffeine + serine which is administered after ethanol intake in human (NN), in which Fig. 15A shows a change of a blood ethanol level with time; Fig. 15B shows a change of a blood acetaldehyde level with time; Fig. 15C shows a change of a blood acetate level with time; and Fig. 15D shows a change of a lactate/pyruvate ratio with time (data are shown by mean±SE);
Fig. 17 shows a relieving effect of hangover when caffeine + serine is taken before alcohol drinking in a human, in which Fig. 17A shows a total of scores; Fig. 17B shows the inquiry results of awakening; Fig. 17C headache, Fig. 17D loss of appetite, Fig. 17E upset stomach, and Fig. 17F thirsty;
Fig. 18 shows a relieving effect of hangover when caffeine + serine is taken after alcohol drinking in a human, in which Fig. 18A shows a total of scores; Fig. 18B shows the inquiry results of awakening; Fig. 18C headache, Fig. 18D loss of appetite, Fig. 18E upset stomach, and Fig. 18F thirsty; and
Fig. 19 shows an effect of recovering from hypoglycemia at the time of fatigue by administration of caffeine + serine in mice.

### Best Mode for Carrying Out the Invention

According to one aspect of the invention, there is provided a composition for accelerating alcohol metabolism or relieving fatigue by *in vivo* gluconeogenesis, characterized by comprising an effective amount of caffeine and an effective amount of an amino acid or a peptide selected from the group consisting of serine, tryptophan, serine rich peptides, tryptophan rich peptides, serine/tryptophan rich peptides and mixtures thereof, as active ingredients.

The caffeine used in the present invention includes 3,7-dihydro-1,3,7-trimethyl-1H-purine-2,6-dione, an acid salt thereof and a hydrate thereof. In the specification, they will be collectively referred to as caffeine. The acid salt refers to a salt of a pharmaceutically acceptable acid including an inorganic acid such as hydrochloric acid, sulfuric acid and citric acid; and an organic acid. The hydrate generally refers to a caffeine-hydrate. A commercially available caffeine may be used herein. Examples of the amount of caffeine contained in the composition, composition set and beverage of the present invention include 10 mg or more, 20 mg or more, 30 mg or more, 40 mg or more, 50 mg or more, 60 mg or more, 70 mg or more, 80 mg or more, 90 mg or more, 100 mg or more, 110 mg or more, 120 mg or more, 130 mg or more, 140 mg or more, 150 mg or more, 170 mg or more, 200 mg or more, 230 mg or more, 250 mg or more, 270 mg or more, 300 mg or more, 330 mg or more, 350 mg or more, 400 mg or more, 450 mg or more, and 500 mg or more. A preferable amount of caffeine falls within an acceptable intake range of a human, more specifically, about 500 mg or less in terms of anhydrous caffeine weight, for example, about 10 to 500 mg, about 20 to 500 mg, about 30 to 500 mg, about 40 to 500 mg, preferably about 50 to 500 mg, about 100 to 500 mg, about 150 to 500 mg or about 200 to 500 mg, more preferably, about 50 to 300 mg, and most preferably about 100 to 300 mg (the Japanese Pharmacopoeia, 13th revised edition (1996), published by Hirokawa Shoten; Remington: The Science and Practice of Pharmacy, the 19th edition (1995), Mack Publishing Company, USA). Caffeine is used as a medicament such as a central nervous system stimulant, diuretic, analgesic (migraine), a medicament for hypertensive headaches, and specified as a dangerous drug. The half-lethal dose of caffeine is 5 to 10 g in adult and 3 g in child. However, it is said that intake of 1 g or more of caffeine at a time causes acute intoxication symptoms of the nervous system and the cardiovascular system, such as collapse, dizziness, and arrhythmia. Therefore, caffeine is preferably used in a dose not exceeding the upper limit approved in the Japanese Pharmacopeia, etc.

The phrase "an amount within an acceptable intake range of a human" used herein means the amount not exceeding the maximum caffeine dose approved by the National Institutes of Health in each country. More specifically, the maximum caffeine dose is, for example, 500 mg (Remington: The Science and Practice of Pharmacy, the 19th edition (1995), Mack Publishing Company, USA).

The term "effective amount" used herein means the amount accelerating alcohol metabolism or relieving fatigue by *in vivo* gluconeogenesis. The term "alcohol metabolism" is interchangeably used with the term "ethanol metabolism".

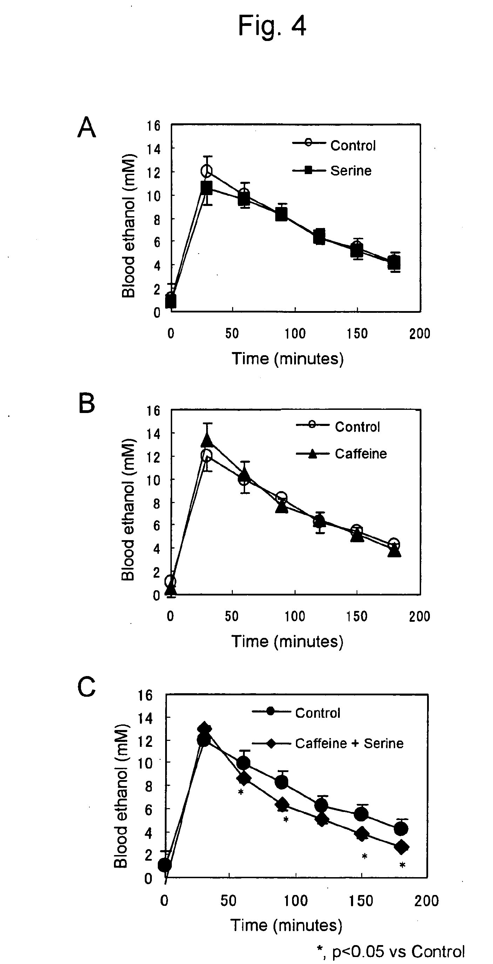

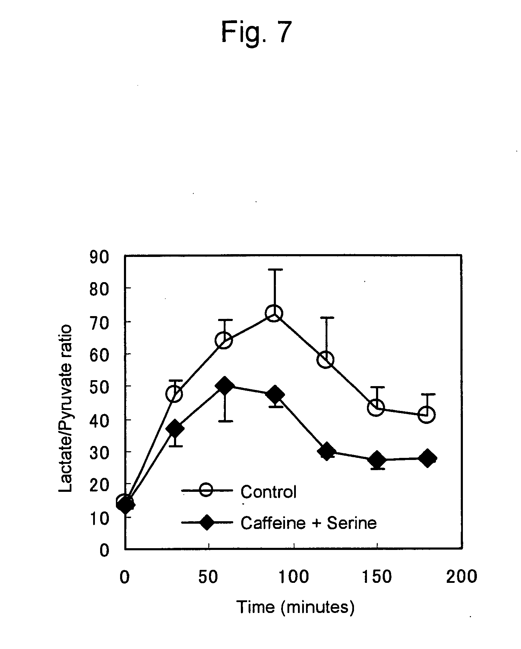

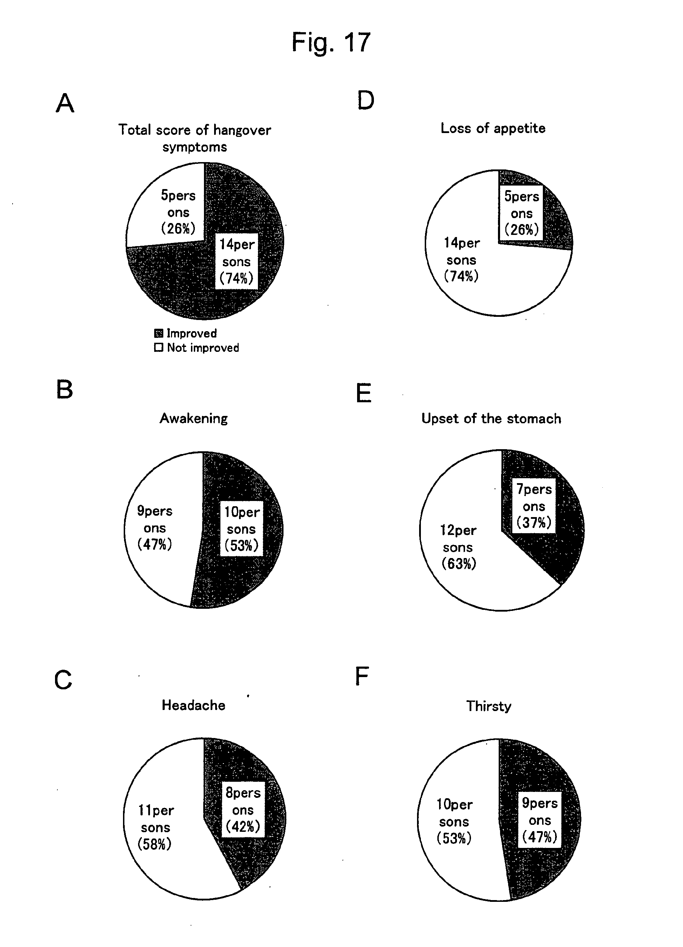

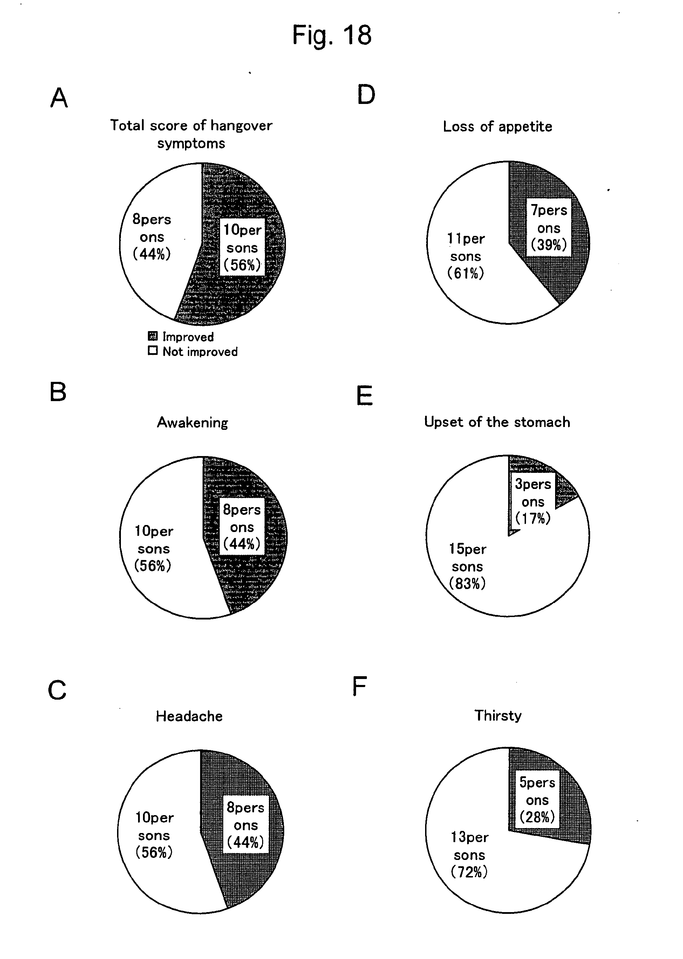

When caffeine is singly taken in an amount within the acceptable intake range, acceleration of alcohol metabolism is not observed (see Fig. 4B). However, when serine or tryptophan is taken in an effective amount in addition to the caffeine in an amount within the acceptable intake range, alcohol metabolism is accelerated and a blood ethanol level decreases either in a hard drinker (genotype ALDH2*2/2 (NN)) or in a light drinker (genotype ALDH2*1/2 (ND)) (see, Figs. 4C, 13A, 15A and 16A). Simultaneously, a blood glucose level increases and a lactate/pyruvate ratio decreases. Therefore, hypoglycemia caused by alcohol drinking is improved and relieving effect on fatigue can be brought (see Figs. 7, 8, 13D, 15D and 16D). As a result, at the time of alcohol intake, comfortable feeling significantly increases. In other words, a physical burden caused by alcohol drinking is zero or mitigated (see Figs. 17 and 18). As shown in Fig. 1, it is presumed that alcohol metabolism and gluconeogenesis are accelerated in concert with each other and the conversion of NADH and NAD⁺ efficiently proceeds, with the result that the alcohol metabolism is accelerated. The term "in concert with each other" used herein refers to the state where the alcohol metabolic pathway and the gluconeogenesis pathway cooperated to accelerate the alcohol metabolic system with coupling.

Accordingly, the composition of the present invention can be used for accelerating alcohol metabolism or relieving a physical burden at the time of alcohol intake (or alcohol drinking), or relieving fatigue. The composition of the present invention can produce the aforementioned effect even if it is taken at any time point of before, during and after intake of alcohol. Furthermore, the composition of the present invention can be formulated into a beverage or food product or a medicament.

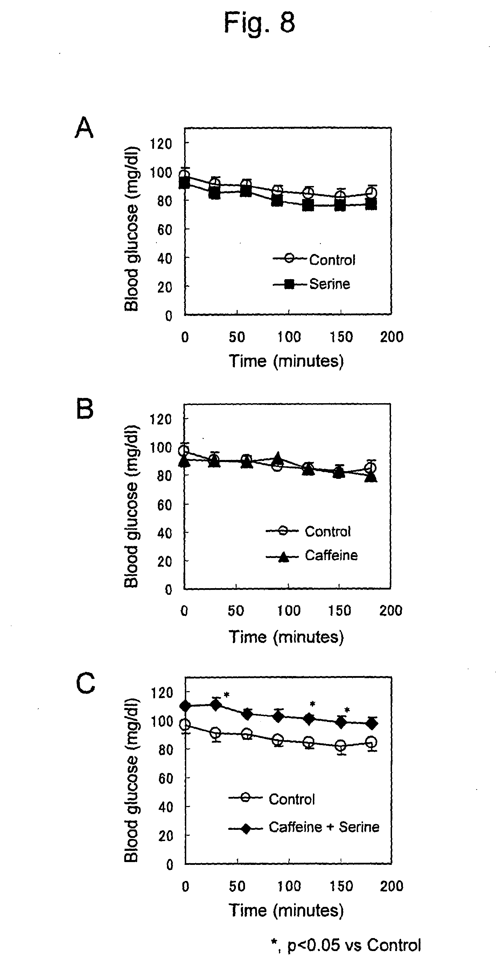

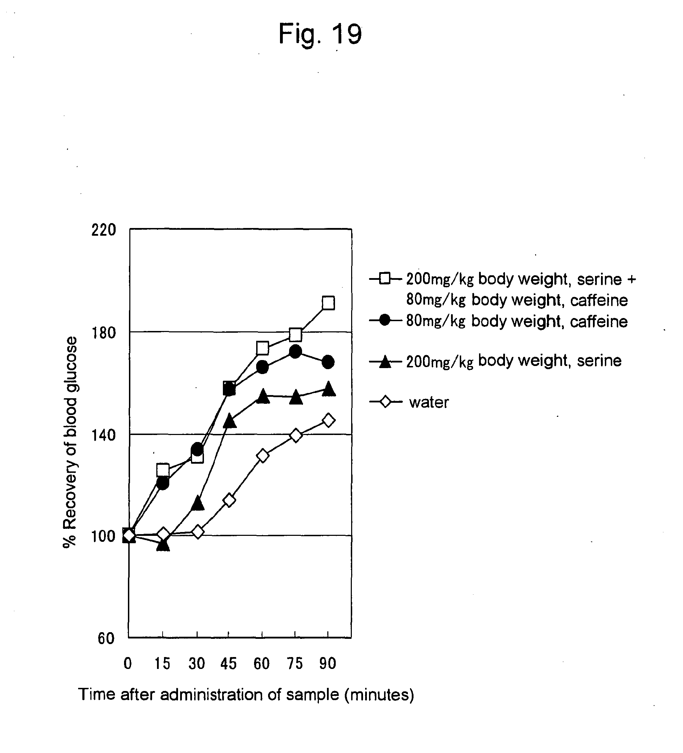

The "alcohol metabolism accelerating" used in the present invention means the metabolism of ethanol into acetate is accelerated without stopping. By virtue of the acceleration of alcohol metabolism, a physical burden such as uncomfortable feeling and collapse caused during and after alcohol drinking can be significantly relieved, remarkably increasing the degree of comfortable feeling. The "relieving fatigue caused by alcohol drinking" means a reduction of the blood lactate level or relieving hypoglycemia, thereby mitigating a physical burden, as shown in the figures showing a reduction of a ratio of lactate /pyruvate in blood (see Figs. 7, 13D, 15D and 16D) and an increase of blood glucose level (see Figs. 8 and 19).

The composition of the present invention contains an effective amount of caffeine and an effective amount of an amino acid or a peptide selected from the group consisting of serine, tryptophan, serine rich peptides, tryptophan rich peptides, serine/tryptophan rich peptides and mixtures thereof.

Serine and tryptophan each is a kind of amino acid and conceivably serves as a substrate of the metabolic system leading to glucose (i.e., gluconeogenesis). In this metabolic system, since it is accompanied by the conversion of NADH to NAD⁺, alcohol metabolism is accelerated without stopping. In an embodiment of the present invention, the composition of the present invention may contain any one of combinations selected from caffeine and serine, caffeine and tryptophan, and caffeine and a mixture of serine and tryptophan. The tryptophan may be free tryptophan or a salt of tryptophan and a pharmaceutically acceptable acid (e.g., tryptophan·HCl). The content of the amino acid or a mixture thereof is not particularly limited as long as it is suitable for significantly and cooperatively accelerating the alcohol metabolism and gluconeogenesis; however, it is, for example, about 100 mg or more, about 200 mg or more, about 300 mg or more, about 400 mg or more, about 500 mg or more, about 600 mg or more, about 700 mg or more, about 800 mg or more, about 900 mg or more, about 1 g or more, about 100 mg to 20 g, about 300 mg to 20 g, about 500 mg to 20 g, about 1 g to 20 g, about 1.5 g to 20 g, about 2 g to 20 g, about 3 g to 20 g, about 4 g to 20 g, about 5 g to 20 g, about 6 g to 20 g, about 7 g to 20 g, about 8 g to 20 g, about 9 g to 20 g, about 10 g to 20 g, about 12 g to 20 g, about 14 g to 20 g, about 16 g to 20 g, and about 18 g to 20 g. However, serine and tryptophan, which are amino acids constituting a protein contained in a beverage or food product and extremely safe, therefore may be taken in an amount exceeding 20 g at a time. The mixing ratio of serine and tryptophan is not particularly limited and any ratio is acceptable. Serine and tryptophan are commercially available, and may be used as they are in the composition of the present invention.

In the present invention, use may be made of a peptide containing a serine residue and/or a tryptophan residue at a high ratio (e.g., about 10% to 100%, about 20% to 100%, about 25% to 100%, about 30% to 100%, about 40% to 100%, about 50% to 100%, about 70% to 100%, about 80% to 100%, or about 90% to 100%) such as serine rich peptide, tryptophan rich peptide, and serine/tryptophan rich peptide. Examples of such a peptide include an oligopeptide, polypeptide, protein and a hydrolysis product or protease digestion product of a protein containing serine and/or tryptophan residue in a high ratio. Such a peptide and a hydrolysis product or protease digestion product of a protein may be enzymatically decomposed into up to amino acids in the digestive tract or after absorbed into the body (in particular, within the blood). Examples of the peptides to be used in the present invention include, but are not limited to, dipeptide, oligopeptide such as tripeptide and tetrapeptide, polypeptide, protein, and proteolytic product, preferably oligopeptide, and more preferably, dipeptide. Examples of the dipeptide include seryl-serine (Ser-Ser), tryptophanyl-tryptophan(Trp-Trp), seryl-tryptophan (Ser-Trp), and tryptotphanyl-serine (Trp-Ser). Examples of the serine-rich protein include sericin (derived from a silkworm). As a peptide to be used in the present invention, a commercially available peptide may be used. Alternatively, a peptide to be used in the invention may be prepared by a conventional peptide synthesis method, a extraction/purification method from a naturally occurring product, genetic engineering, and enzymatic proteolysis (for example, new biochemical experiment course 1, Vols. I, V and VI, and new biochemical experimental course 2, Vol. III, edited by the Japanese biochemical society,) singly or in appropriate combination thereof. The content of a peptide in the composition of the present invention is not particularly limited, however, it is, for example, about 100 mg or more, about 200 mg or more, about 300 mg or more, about 400 mg or more, about 500 mg or more, about 600 mg or more, about 700 mg or more, about 800 mg or more, about 900 mg or more, about 1 g or more, about 100 mg to 20 g, about 300 mg to 20 g, about 500 mg to 20 g, about 1 g to 20 g, about 1.5 g to 20 g, about 2 g to 20 g, about 3 g to 20 g, about 4 g to 20 g, about 5 g to 20 g, about 6 g to 20 g, about 7 g to 20 g, about 8 g to 20 g, about 9 g to 20 g, about 10 g to 20 g, about 12 g to 20 g, about 14 g to 20 g, about 16 g to 20 g, about 18 g to 20 g, or about 20 g or more. The content of a peptide in terms of a total weight of free serine and/or free tryptophan, is about 100 mg or more, about 200 mg or more, about 300 mg or more, about 400 mg or more, about 500 mg or more, about 600 mg or more, about 700 mg or more, about 800 mg or more, about 900 mg or more, or about 1 g or more.

According to a preferable embodiment of the present invention, the content of serine in the composition of the present invention is 500 mg or more per composition in terms of free serine.

According to another preferable embodiment of the present invention, the content of serine in the composition of the present invention is 1 g or more per composition in terms of free serine.

According to another aspect of the present invention, there is provided a fatigue relieving composition characterized by comprising an effective amount of caffeine and an effective amount of an amino acid or a peptide selected from the group consisting of serine, tryptophan, serine rich peptides, tryptophan rich peptides, serine/tryptophan rich peptides and mixtures thereof, as active ingredients.

According to an embodiment of the present invention, there is provided a fatigue relieving composition comprising an effective amount of caffeine and an effective amount of serine as active ingredients. The content and type of the amino acid and caffeine serving as active ingredients are the same as described above. The composition of the present invention, since it contains an amino acid or a peptide and caffeine as active ingredients, is useful in ameliorating hypoglycemia as mentioned above. The composition of the present invention is preferably a beverage or food or a medicament. Fatigue is a biological reaction taking place within the body caused by physical activity, physical exercise, stress, lack of sleep, and a disease (e.g., allergic disorder, infectious disease, cancer etc.). The composition of the present invention can be used for recovering the body from fatigue and relieving fatigue taking place in daily life or mitigating a physical burden (collapse) caused by alcohol intake.

The present invent further provides a composition containing caffeine in an effective amount of about 500 mg or less and a combination consisting of at least 2 components selected from the group consisting of serine or serine rich peptides, tryptophan or tryptophan rich peptides, and serine/tryptophan rich peptides, in an effective amount of about 100 mg or more. The use of the composition is not particularly limited; however, the composition may be used as a beverage or food or a medicament. The content and type of the amino acid and caffeine serving as active ingredients are the same as described above. Examples of the compositions include a composition containing caffeine and a mixture of serine and tryptophan, a composition containing caffeine and a mixture of serine and a tryptophan-rich peptide (e.g., Trp-Trp dipeptide), a composition containing caffeine and a mixture of tryptophan and serine-rich peptide (e.g., Ser-Ser dipeptide), a composition containing caffeine and a mixture of serine and a serine/tryptophan rich peptide (e.g., Ser-Trp or Trp-Ser dipeptide), or a mixture of tryptophan and a serine/tryptophan rich peptide (e.g., Ser-Trp or Trp-Ser dipeptide). The total amount of an amino acid and a peptide as mentioned above per composition is about 100 mg or more, about 200 mg or more, about 300 mg or more, about 400 mg or more, about 500 mg or more, about 600 mg or more, about 700 mg or more, about 800 mg or more, about 900 mg or more, and about 1 g or more, more specifically, about 100 mg to 20 g, about 300 mg to 20 g, about 500 mg to 20 g, about 1 g to 20 g, about 1.5 g to 20 g, about 2 g to 20 g, about 3 g to 20 g, about 4 g to 20 g, about 5 g to 20 g, about 6 g to 20 g, about 7 g to 20 g, about 8 g to 20 g, about 9 g to 20 g, about 10 g to 20 g, about 12 g to 20 g, about 14 g to 20 g, about 16 g to 20 g, about 18 g to 20 g, or about 20 g or more, in terms of a total weight of free serine and/or free tryptophan. Furthermore, the amount of caffeine per composition preferably falls within an acceptable intake range of a human, more specifically, about 500 mg or less, for example, about 10 to 500 mg, about 20 to 500 mg, about 30 to 500 mg, about 40 to 500 mg, preferably about 50 to 500 mg, more preferably, about 50 to 300 mg, and, most preferably, about 100 to 300 mg.

According to a preferable embodiment of the present invention, the content of serine contained in a combination as mentioned above is 100 mg or more per composition in terms of free serine.

According to another preferable embodiment of the present invention, the content of serine contained in a combination as mentioned above is 500 mg or more per composition in terms of free serine.

According to still another preferable embodiment of the present invention, the content of serine contained in a combination as mentioned above is 1 g or more per composition in terms of free serine.

The present invention further provides a composition containing caffeine (500 mg or less) and a serine/tryptophan rich peptide (about 100 mg or more). The caffeine and serine/tryptophan rich peptide are the same as defined above. The type and content of them are the same as mentioned above. In particular, the serine/tryptophan rich peptide contains serine and tryptophan residues in a ratio of about 10% or more to about 100% in the amino acid composition of the peptide, and includes an oligopeptide, polypeptide, protein and a hydrolysis product or protease digestion product of the protein. The total amount of serine/tryptophan rich peptide per composition in terms of a total weight of free serine and/or free tryptophan is about 100 mg or more, about 200 mg or more, about 300 mg or more, about 400 mg or more, about 500 mg or more, about 600 mg or more, about 700 mg or more, about 800 mg or more, about 900 mg or more, about 1 g or more, more specifically, about 100 m g to 20 g, about 300 m g to 20 g, about 500 m g to 20 g, about 1 g to 20 g, about 1.5 g to 20 g, about 2 g to 20 g, about 3 g to 20 g, about 4 g to 20 g, about 5 g to 20 g, about 6 g to 20 g, about 7 g to 20 g, about 8 g to 20 g, about 9 g to 20 g, about 10 g to 20 g, about 12 g to 20 g, about 14 g to 20 g, about 16 g to 20 g, about 18 g to 20 g, or about 20 g or more. Furthermore, the amount of caffeine per composition preferably falls within an acceptable intake range of a human, more specifically, about 500 mg or less, for example, about 10 to 500 mg, about 20 to 500 mg, about 30 to 500 mg, about 40 to 500 mg, preferably, about 50 to 500 mg, more preferably, about 50 to 300 mg, and most preferably, about 100 to 300 mg. The composition can also be formulated into a beverage or food or a medicament.

The present invention provides an alcohol metabolism accelerating composition characterized by comprising an effective amount of caffeine and an effective amount of an amino acid or a peptide selected from the group consisting of serine, tryptophan, serine rich peptides, tryptophan rich peptides, serine/tryptophan rich peptides and mixtures thereof, as active ingredients. In an embodiment thereof, the composition of the present invention can contain caffeine, serine and tryptophan or a mixture thereof as active ingredients. Furthermore, the composition is a beverage or food or a medicament.

According to a preferable embodiment of the present invention, the content of serine contained in the composition of the present invention is 500 mg or more per composition in terms of free serine.

According to another preferable embodiment of the present invention, the content of serine contained in the composition of the present invention is 1 g or more per composition in terms of free serine.

The present invention further provides a composition set containing a first composition containing caffeine in an effective amount of about 500 mg or less and a second composition containing serine, tryptophan, or a mixture thereof in an effective amount of about 100 mg or more in combination as discrete packages. Examples of the composition set of the present invention include a beverage or food set having coffee or tea (including beverage thereof) containing caffeine and sugar containing serine or tryptophan in combination, and a medicament set having a caffeine containing preparation and a serine or tryptophan containing preparation in combination.

According to a preferable embodiment of the present invention, the content of serine contained in a second composition as mentioned above is 500 mg or more.

According to another preferable embodiment of the present invention, the content of serine contained in a second composition as mentioned above is 1 g or more.

The present invention further provides a caffeine containing beverage characterized by containing 500 mg or more of serine per beverage.

The beverage of the present invention is not particularly limited as long as it contains caffeine; however, may be selected from the group consisting of green tea, black tea, coffee, cola and a drinkable preparation.

According to a preferable embodiment of the present invention, the content of caffeine is 50 mg to 500 mg (both inclusive), 100 mg to 500 mg (both inclusive) or 150 mg to 500 mg (both inclusive).

The composition of the present invention may take forms of, but not limited to, a beverage or food and a medicament.

Examples of the beverage or food include conventional or novel beverages or foods such as processed grain products (e.g., bread and rice cake), confectioneries (e.g., chocolate, gum, candy, western confectionery, Japanese confectionery, snack, rice confectionery), beverages (e.g., drinkable preparations, alcohol drink including distilled spirit highball and cocktail, etc.), non-alcohol beverage such as coffee, black tea, green tea, oolong tea, cola, guarana beverage, sport beverage, juice, amino acid beverage, soda beverage, turmeric tea), dairy products (e.g., cheese, butter, milk and ice cream etc.), meat products (e.g., ham, sausage), fish products (e.g., steamed fish paste, fish sausage), supplements and health foods, but not limited these. Examples of preferable beverages or foods include drinking preparations, non-alcohol beverages and supplements.

When employed in a supplement and a health food, the composition of the present invention may further contain at least one component selected from the group consisting of an amino acid other than serine and tryptophan, such as alanine, glycine, lysine, leucine, glutamine, cysteine, proline, and L-theanine, turmeric, sesamin, milk-thistle, Densiti carrot (Chinese ginseng) and oyster extract, etc.

During a manufacturing step of each beverage or food, caffeine, and serine, tryptophan, a serine rich peptide, a tryptophan rich peptide, a serine/tryptophan rich peptide or mixtures thereof may be mixed with or added to the beverage or food within the aforementioned dose range in combination with a known substance useful for a human body, under the conditions where the active ingredients are not changed.

The composition and beverage of the present invention may further contain a carrier, dilution, excipient, expander, gelatinization agent, dispersant, suspending agent and auxiliary solubilizer, which are generally contained in beverages or foods, and additionally, a vitamin, organic acid, sweetening agent, pH regulator, flavor agent, coloring agent, stabilizer, mineral, spice, preservative, humectant, fruit juice, milk and sweetening agent.

The content of the active ingredient contained in the composition and the beverage of the present invention is as described above. For example, a drinkable preparation having a volume such as 30 ml, 50 ml, and 100 ml per bottle contains caffeine in an effective amount of about 500 mg or less and an amino acid or a peptide in an effective amount of about 100 mg or more in admixture of a diluent and additive(s).

Furthermore, the beverage may be manufactured as a so-called "a product in container" prepared by charging the composition of the present invention in a container and sealing it. Such a beverage may be manufactured by a method including a step of preparing the composition of the present invention, a step of sterilizing the obtained composition, and a step of charging the sterilized composition to a container, followed by sealing. The material for the container may include, but is not limited to, a metal such as steel and aluminium, polymer such as PET, and glass. The shape of the container may be a bottle or a can, etc. Examples of the sealing material include a screwed cap and a cover cap, etc. Sterilization may be performed by any one of sterilization methods generally used in the beverage industry, for example, heat sterilization, filter sterilization, and UV sterilization.

Note that the types, forms and manufacturing methods of beverages are described in "Soft drinks update" edited by update soft drinks editor committee, published by Kohrin, October 2003 (Tokyo, Japan). They are available for the present invention. The types, forms and manufacturing methods of tablets and granules used as supplements are described for example in "Tablets" edited by Shunich Naito, published by Hirokawa Shoten (July, 1990) (Tokyo Japan); and "Granulation handbook" edited by the Association of Powder Process Industry and Engineering, published by Ohmsha (March 1991)(Tokyo, Japan). They are available for the present invention.

Examples of a medicament include an alcohol metabolism accelerator or a physical burden mitigating agent or a medicament for relieving fatigue (or a fatigue refresher). Examples of preparations include tablets, pills, capsules, powders, granules, emulsions, solutions, suspensions and syrups. Additionally, these preparations may contain pharmaceutically acceptable additives such as a carrier, excipient, diluent, preservative, humectant, emulsifier, dispersant, stabilizer, disintegrator, coloring agent, flavor and spice. The medicament of the present invention serving as a fatigue refresher may contain a known agent such as a vitamin (e.g., vitamin B1), a Chinese medicine (e.g., Korean ginseng), mineral (e.g., Ca, Mg, Zn, Fe), royal jelly, carnitine, coenzyme Q10, and taurine, etc. In particular, to mitigate a physical burden after alcohol drinking, the medicament may contain at least one component selected from the group consisting of an amino acid other than serine and tryptophan, such as alanine, glycine, lysine, leucine, glutamine, cysteine, proline, and L-theanine, turmeric, sesamin, milk-thistle, Densiti carrot (Chinese ginseng) and oyster extract, etc. Examples of a preferable form of the medicament include a liquid charged in a small-volume bottle container, a so-called drinkable preparation. As an administration method, an oral administration method or a parenteral administration may be mentioned; however an oral administration method is preferably employed. The effective amount of an active ingredient per preparation is as described above. In particular, according to the Japanese Pharmacopoeia (the 13th revised, 1996, Hirokawa Shoten) the effective amount of caffeine is about 100 to 300 mg per time per human adult and the total dose per day is desirably 500 mg or less.

The present invention will be described in more detail below by way of Examples, which should not be construed as limiting the present invention.

### Examples

### <Example 1>

### Metabolic change of a rat by administration of caffeine

To sixth week-old male Wistar rats (Charles River Laboratories Japan, Inc., Yokohama, Japan), 100 mg/kg body weight of caffeine (anhydrous caffeine, Wako Pure Chemical Industries, Ltd. Osaka, Japan) or water (as control) was orally administered. Two hours later, the rats were anesthetized with ether. Blood was taken from the aorta abdominalis and the liver was excised out. From the blood, the plasma was immediately prepared and a glucose level was measured by use of glucose CII Test Wako (Wako Pure Chemical Industries Ltd.). On the other hand, the liver was frozen with liquid nitrogen immediately upon excising. Total RNA was prepared from about 50 mg of the liver tissue by TRIzol (Invitrogen) and purified by RNeasy Mini (Quiagen) and RNase-Free DNase Set (Quiagen). From the total RNA (5 µg) thus purified, cDNA was synthesized by use of Thermo Script RT-PCR system (Invitrogen) and the mRNA expression levels of various genes were determined by FastStart DNA Master SYBR Green I (Roche) and lightCycler (Roche). The expression level of each gene was corrected based on the expression level of a housekeeping gene P0 to calculate a relative expression level. The DNA primers used in the gene expression analysis were: 5-' acgctgccgtcaaggaaggaaagc-3' "SEQ ID NO: 1" and 5'-gcgggctccaccaggatcttctcat-3' "SEQ ID NO: 2", as sense and antisense primers of serine dehydratase (SDS); 5'-gctgctgaaatcggagcaggagca-3' "SEQ ID NO: 3" and 5'-ctgcttccggaactctgccatctg-3' "SEQ ID NO: 4", as sense and antisense primers of tryptophan2,3-dioxygenase (TDO); 5'-tcgaggccgtaatccgctttgc-3' "SEQ ID NO: 5" and 5'-gaaacccgcactcgcccatgta-3' "SEQ ID NO: 6", as sense and antisense primers of alanine-amino transferase (ALT1); and 5'-ggggaacgtgggctttgtgttca-3' "SEQ ID NO: 7" and 5'-tggtgatgcccaaagcttggaaga-3' "SEQ ID NO: 8", as sense and antisense primers of house keeping gene acidic ribosomal phosphoprotein P0. These primers were all synthesized by Invitrogen.

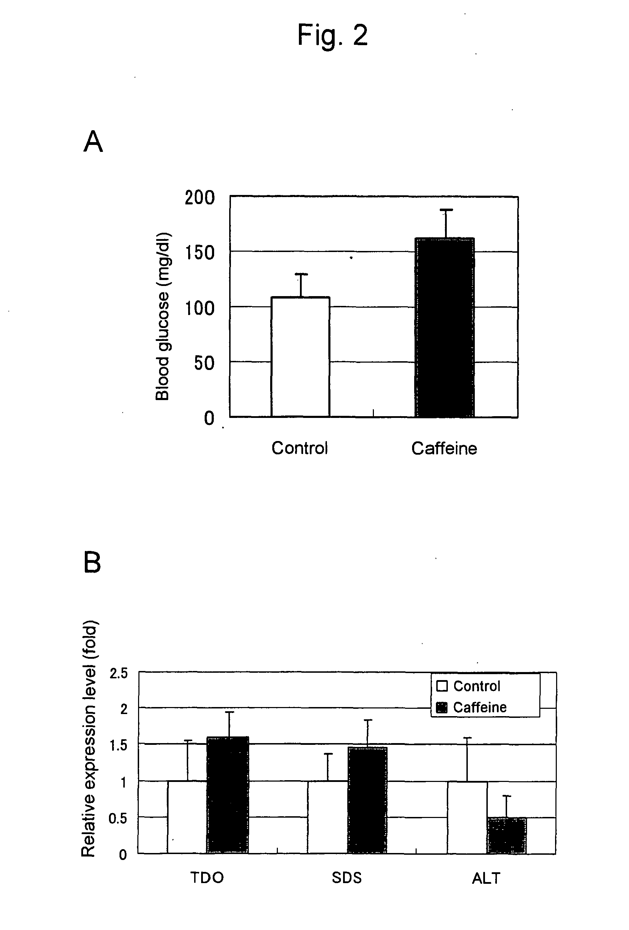

As shown in Fig. 2A, it was found that caffeine has an effect of increasing a blood glucose level, in other words, an effect of accelerating gluconeogenesis. It is said that the mammalian gluconeogenesis takes place in the liver in which glucose is biologically synthesized from an amino acid through a complicated metabolic pathway (Biochemistry, Zubay, published by Hirokawa Shoten). Then, a change of the gene expression of SDS and TDO was analyzed. As a result, it was suggested that the metabolism of serine and tryptophan was enhanced (Fig. 2B). On the other hand, the alanine metabolism pathway (ALT1), which was generally said to play a principal role in gluconeogenesis taking place in the liver, was not enhanced. From this fact, it was suggested that, the gluconeogenesis accelerated by caffeine is principally related to the metabolism of serine and tryptophan unlike a general gluconeogenesis.

### <Example 2>

### Reduction of blood ethanol level by administration of caffeine + serine to rat

A cannula was surgically inserted into the cervical vein of male Wistar rats (Japan SLC, Hamamatsu, Japan) having a body weight of 250 g to 300 g in order to take blood with time under no anesthesia. Four groups were prepared in total as follow. A control group of rats (n=6) was prepared by orally administering water. A group of rats (n=7) was prepared by administering 20 mg/kg body weight of caffeine. A group of rats (n=5) was prepared by administering 0.1 g/kg body weight of serine. A group of rats (n=5) prepared by administering 20 mg/kg body weight of caffeine and 0.1 g/kg body weight of serine. Since it has been said that when 1 g or more of caffeine is taken at a time, acute intoxication symptoms of the nervous system and the cardiovascular system, such as collapse, dizziness, and arrhythmia, arise. Therefore, the dose of caffeine was set within the range whose upper limit is 20 mg/kg body weight, at which acute intoxication symptom presumably appears in an average adult female having a body weight of 50 kg. On the other hand, serine was set at 0.1 g/kg body weight which is sufficiently lower than the acute half lethal dose (14 g/kg body weight) in a rat. One hour after administration with individual samples, ethanol (2 g/kg body weight) was orally administered. Blood was taken from the cervical vein 1, 2, 3 and 5 hours after the administration. Immediately upon taking, the blood was mixed well with a solution containing 0.6N perchlorate and 0.9% NaCl, and centrifugally separated at 3000 rpm for 20 minutes to remove protein. The ethanol concentration of the supernatant was measured with a gas chromatograph manufactured by Shimazu Corporation (Eriksson CJP et al., Anal. Biochem. Vol.125, 259-263 (1982)).

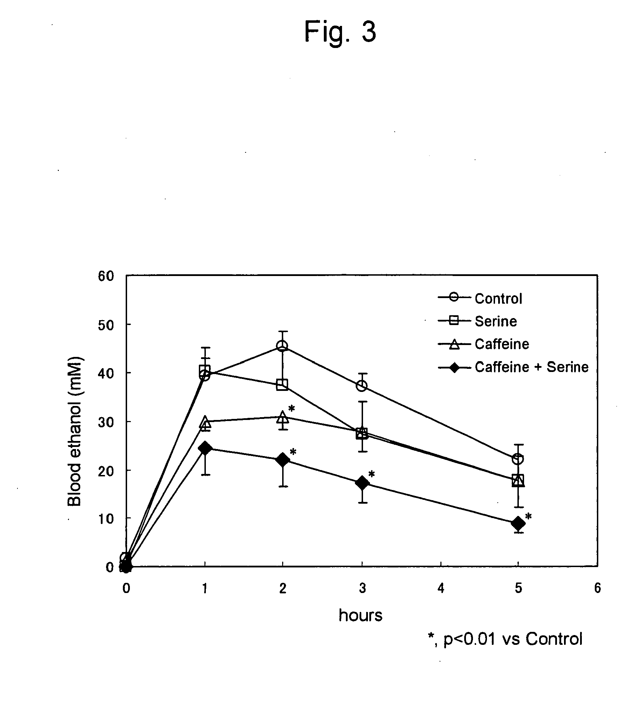

A change of blood ethanol is shown in Fig. 3. It was found that the blood ethanol level of the serine administration group slightly decreased compared to the control group; however, this change observed in the all measurement time points had not a significance. In the caffeine administration group, the blood ethanol level was observed to slightly decrease and significantly decreased 2 hours after ethanol administration; however, a significant effect was not observed in other time point. In contrast, in the group to which caffeine and serine were both administered simultaneously, a significant decrease of blood ethanol level was observed. More specifically, a significant effect was observed in all measurement time points on and after 2 hours after ethanol was administered. Based on these results, it was deduced that ethanol metabolism is not sufficiently accelerated even if 0.1 g/kg body weight of serine is taken and even if caffeine is taken in a high concentration at which acute intoxication may occur, sufficient acceleration of the ethanol metabolism may not be expected. On the other hand, when caffeine and serine both are taken simultaneously, the blood ethanol level significantly reduced, demonstrating that the ethanol metabolism is clearly accelerated.

### <Example 3>

### Acceleration of ethanol metabolism by caffeine + serine intake in human

Eight healthy adults who had agreed to participate in the test before the test began in view of ethical consideration and had a genotype of acetaldehyde dehydrogenase (ALDH2): ALDH2*1/2 (ND) were chosen as subjects for the test. The subjects were asked to have no food and water from wake-up time to participate in the test. A control test was performed by 8 persons (as volunteers). Of the 8 persons participated in the control test, 5 persons were chosen to perform a sample administration test. As a sample, 3 mg/kg body weight of caffeine (manufactured by SHIRATORI Pharmaceutical Co., Ltd., Chiba Japan) alone, 30 mg/kg body weight of serine (manufactured by KYOWA HAKKO KOGYO Co., Ltd. Tokyo, Japan) alone, or 3 mg/kg body weight of caffeine and 30 mg/kg body weight of serine were orally administered. The amount of caffeine was set based on the amount taken by a human having body weight of 50 kg from a cup of coffee (about 150 mg), which may not cause acute intoxication. The amount of serine, which was significantly low compared to an acute half-lethal dose (14 g/kg body weight) of a rat, was set on the assumption that 40% of an average human intake amount (3.71 g) per day is taken by an average human having a body weight of 50 kg. One hour after the sample intake, 5% ethanol solution (white liquor diluted with water) was divided into 6 portions and orally taken 6 times at the intervals of 4 minutes so as to take 0.4 g ethanol per a body weight (kg). Blood was taken immediately before alcohol drinking, 30, 60, 90, 120, and 180 minutes after alcohol drinking. Immediately upon taking blood, blood was mixed with a solution containing 0.6N perchlorate and 0.9% NaCl, and centrifugally separated at 3000 rpm for 20 minutes to remove protein. The ethanol, acetaldehyde and acetate concentrations of the supernatant were measured by gas chromatography manufactured by Perkin Elmer Corporation or Shimazu Corporation (Eriksson CJP et al., Anal. Biochem. Vol.125, 259-263 (1982)). In addition, immediately upon taking blood, the plasma was prepared from part of the blood and blood glucose level was measured by use of glucose CII TestWako (Wako Pure Chemical Industries, Ltd.). Furthermore, using the same plasma, blood lactate and pyruvate levels were measured by use of determiner LA (Kyowa Medics, Tokyo, Japan) and determiner PA (Kyowa Medics), respectively. Based on the ratio of lactate/pyruvate, the ratio of NADH/NAD⁺ was calculated. On the other hand, at the same time of taking blood, 9-rank subjective evaluation was performed by the subjects with respect to "feeling of drunk" (by scores 0 to 8) and comfortable feeling (uncomfortable (score -4) to comfortable (score 4)).

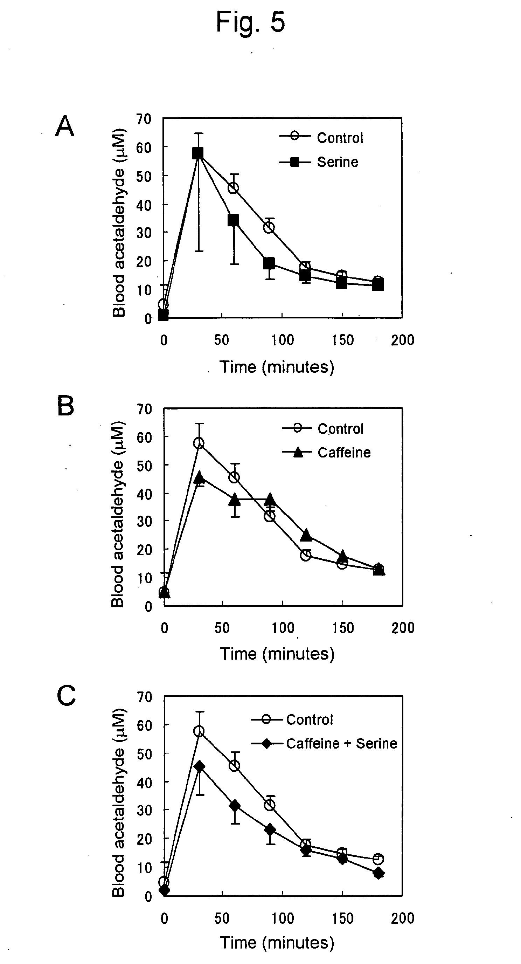

As shown in Fig. 4, the blood ethanol level rarely changed when serine alone or caffeine alone was taken; however, significantly reduced on and after 60 minutes after caffeine + serine was taken. On the other hand, the blood acetaldehyde level did not significantly change in any one of the samples of single intake of serine, single intake of caffeine and caffeine + serine intake. However, the blood acetaldehyde level of the caffeine + serine intake case always goes at a lower level than the control unlike other two cases. From this, it was conceivable that the caffeine + serine has a tendency to lower the acetaldehyde level (Fig. 5). From the results above, it is difficult to sufficiently reduce the blood ethanol level and acetaldehyde level when serine or caffeine is singly taken at an intake level determined in consideration of eating experience and safeness. Thus, it was found for the first time that substantial effect is produced by taking caffeine and serine in combination.

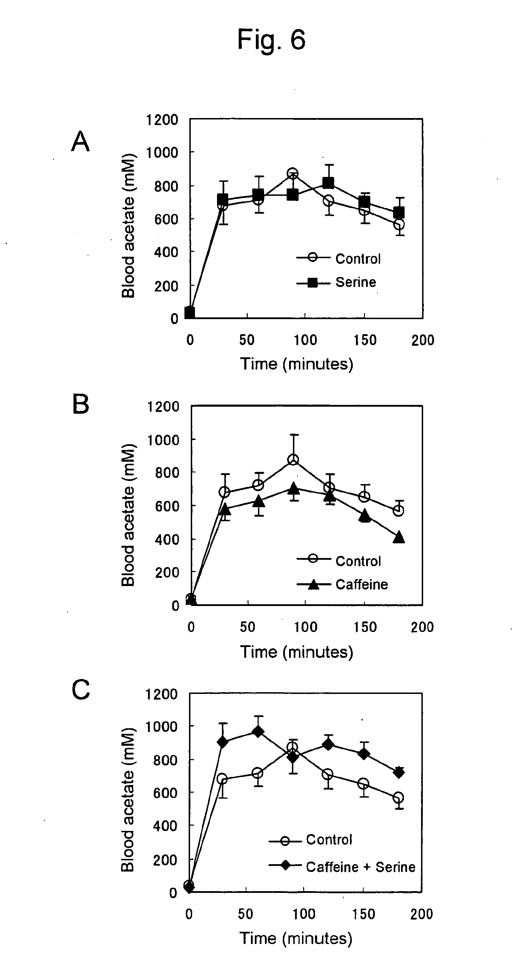

When serine + caffeine were taken in combination, a maximum value of blood ethanol level was the same as in the control 30 minutes after the ethanol intake and the reduction of the blood ethanol level was observed on and after 60 minutes. From this, it was deduced that the reduction of the blood ethanol level is not due to inhibition of ethanol absorption but acceleration of ethanol metabolism. As described above, ethanol is metabolized into acetaldehyde by ADH, which is then metabolized into acetate by ALDH. Since the intake of caffeine + serine reduces the blood ethanol level, the reaction from ethanol to acetaldehyde by ADH is conceivably accelerated. However, if only the ADH reaction proceeds, the production amount of acetaldehyde would be equal to or larger than that of the control. However, the blood acetaldehyde level showed a downward tendency as well. From this fact, it was considered that the metabolism from acetaldehyde into acetate might be elevated. Then, the blood level of an acetaldehyde metabolized product, i.e. acetate, was measured. The blood acetate level of a caffeine + serine intake case showed an upward tendency compared to the control (Fig. 6). It was suggested that the reaction from ethanol to acetate might be elevated without stopping.

ADH and ALDH reactions are common in the respect of requiring NAD⁺. Therefore, it is known that the ethanol metabolism slows down when the ratio of NADH/NAD⁺ increases. The ratio of NADH/NAD⁺ *in vivo* can be calculated based on the ratio of lactate/pyruvate in the blood. Therefore, the ratio of lactate/pyruvate in the blood of caffeine +serine intake was measured. As a result, it was found that the degree of an increase by the ethanol intake is lower than the control (Fig. 7). When caffeine + serine is taken, the blood glucose level goes at a higher value than in the control (Fig. 8). It was estimated that the conversion of NADH to NAD⁺ in the gluconeogenesis metabolism system contributed to acceleration of ethanol metabolism.

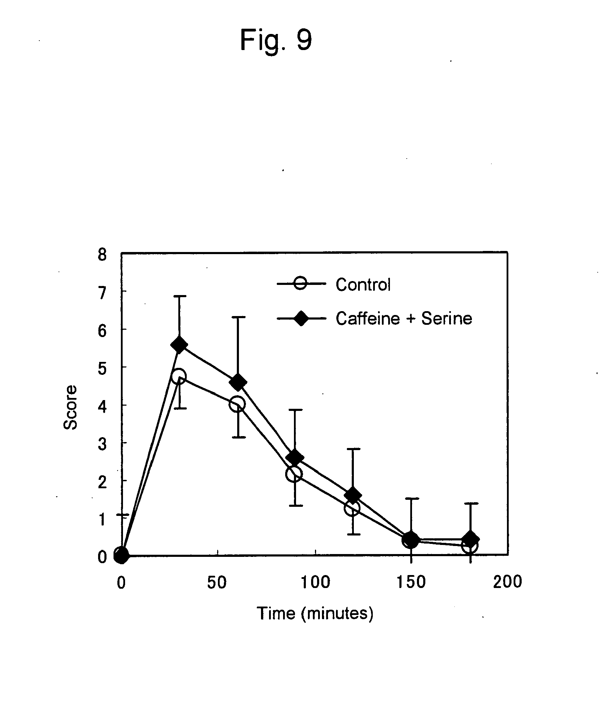

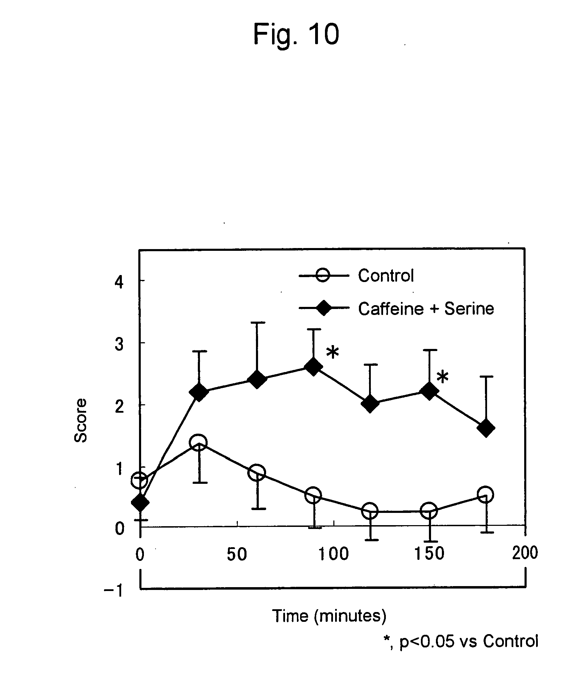

Subjective evaluation results are shown in Figs. 9 and 10. From this, it was clearly demonstrated that when caffeine + serine was taken, no significant change is given to "feeling drunk", but "comfortable feeling" significantly increases. From this, it is considered that intake of caffeine + serine may be effective in relieving uncomfortable feeling that a light drinker has in alcohol drinking.

### <Example 4>

### Reduction of blood ethanol level in rat by administration of caffeine + tryptophan

Using male Wistar rats (Japan SLC) having a body weight of 250 g to 300 g, four groups were set in total: a control group of rats (n=4) prepared by orally administering water; a group of rats (n=9) prepared by administering 20 mg/kg body weight of caffeine (Wako Pure Chemical Industries, Ltd), a group of rats (n=6) prepared by administering 0.1 g/kg body weight of tryptophan (Wako Pure Chemical Industries, Ltd) and a group of rats (n=7) prepared by taking 20 mg/kg body weight of caffeine and 0.1 g/kg body weight of tryptophan. One hour later, ethanol was administered. After further two hours, blood was taken from the aorta abdominalis under anesthesia with pentobarbital (Sigma). The blood ethanol level was measured in the same manner as in Example 2.

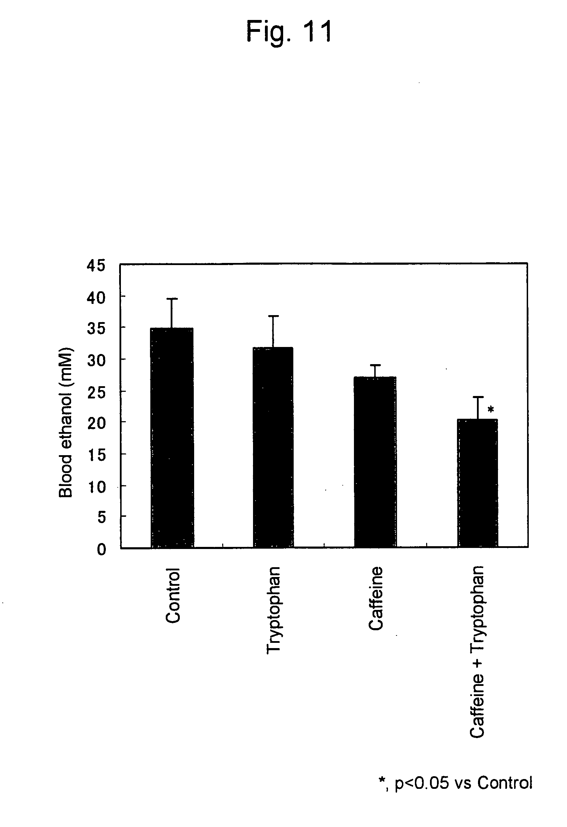

The results of blood ethanol level are shown in Fig. 11. No change was observed in the tryptophane single administration group. A slight downward tendency was observed in the caffeine administration group,, but it is not significant change. In contrast, a significant reduction of blood ethanol level was observed in a caffeine + tryptophan administration group.

### <Example 5>

### Change of blood ethanol in rat by administration of caffeine + each of various amino acids

Using male Wistar rats (Japan SLC) having a body weight of 250 g to 300 g, 22 groups were set in total: a control group of rats (n=4) prepared by orally administering water; an amino acid free group of rats (n=4) prepared by administering 20 mg/kg body weight of caffeine (Wako Pure Chemical Industries, Ltd) alone, and 20 groups of rats (n=2 per group) prepared by taking 20 mg/kg body weight of caffeine and 0.1 g/kg body weight of each of 20 types of amino acids. One hour after the administration of each sample, ethanol (2 g/kg body weight) was orally administered. After further two hours, blood was taken from the aorta abdominalis under anesthesia with pentobarbital (Sigma). The blood ethanol level was measured by gas chromatography manufactured by Shimazu Corporation in the same manner as in Example 2.

Fig. 12 shows relative blood ethanol levels of amino acid administration groups compared to the group to which only caffeine was administered (i.e., the amino acid free group), which is regarded as 1. The blood ethanol reduction effect was observed in the serine administration group and the tryptophan administration group compared to the amino acid free group; however, not observed in other amino acid addition groups. From the results, it was demonstrated that serine or tryptophan is preferable as an amino acid for producing effect by taking it together with caffeine.

### <Example 6>

### Acceleration of ethanol metabolism by caffeine + serine intake in human (NN)

Continued from the test directed to a relatively light drinker (having an acetaldehyde dehydrogenase (ALDH2) genotype of ND) performed in Example 3, the ethanol-metabolism accelerating effect upon a hard drinker (ALDH2*2/2 (NN)) was checked. Five healthy adults having a genotype of NN were selected as subjects for the test after obtaining informed consent. When a test was performed in the morning, they were asked to take neither meal nor water from the wake-up time to participate in the test. When a test was performed in the afternoon, the subjects were asked to take neither meal nor water after taking breakfast. As a sample, 3 mg/kg body weight of caffeine (manufactured by SHIRATORI Pharmaceutical Co., Ltd.) and 30 mg/kg body weight of serine (KYOWA HAKKO KOGYO Co., Ltd.) were orally taken. One hour after the intake, 5% ethanol solution was further orally given so as to be 0.4 g/kg body weight of ethanol. Blood was taken immediately before alcohol intake, 30, 60, 90, 120 and 180 minutes after the alcohol intake, in the same manner as in Example 3. The ethanol, acetaldehyde and acetate levels were measured by gas chromatography. Furthermore, part of the blood was subjected to measurement of lactate level and pyruvate level. The ratio of NADH/NAD⁺ was calculated from the ratio of lactate/pyruvate.

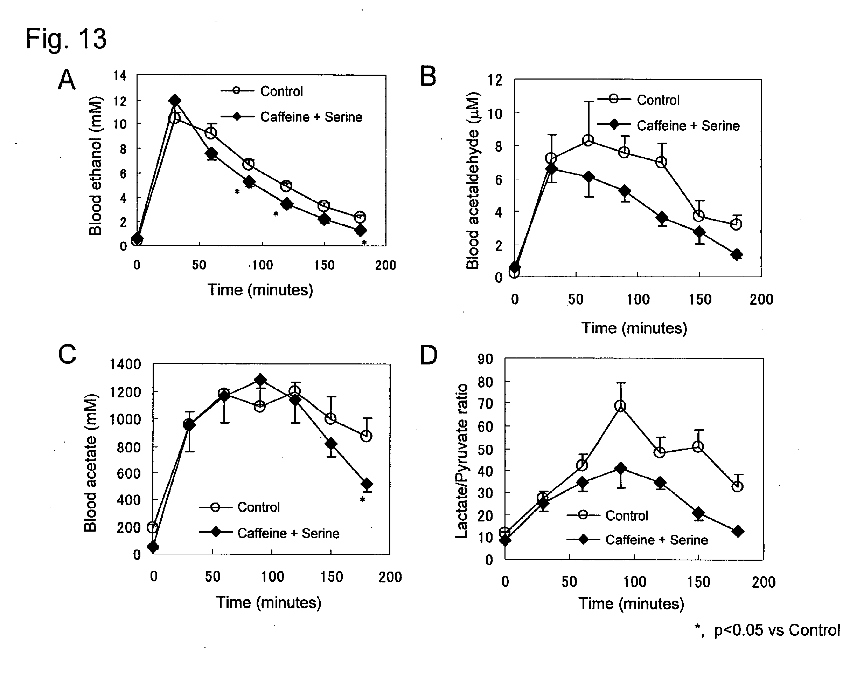

As shown in Fig. 13A, the blood ethanol level showed a downward tendency from 60 minutes after the ethanol intake by caffeine + serine intake. A significant reduction was observed at 90, 120 and 180 minutes after the intake. The blood ethanol level was not reduced compared to the control at 30 minutes after the ethanol intake. From this fact, it was conceivable that the reduction of the blood ethanol level observed above is not due to inhibition of ethanol absorption but due to acceleration of ethanol metabolism. In addition, a downward tendency of the blood acetaldehyde level was observed on and after 60 minutes by caffeine + serine intake. From this, it was considered that not only the metabolism from ethanol to acetaldehyde by ADH and but also the metabolism from acetaldehyde to acetate by ALDH were accelerated (Fig. 13B). In the subject (NN), the blood acetate level did not increase over in the control. From this, it was demonstrated that a temporal increase of the blood acetate level caused by ethanol intake is terminated earlier by acceleration of ethanol metabolism (Fig. 13C). Furthermore, the ratio of lactate/pyruvate in blood reflecting the ratio of NADH/NAD⁺ was lower than in the control by caffeine +serine intake. It was deduced that the conversion NADH into NAD⁺ caused by the acceleration of the gluconeogenesis system contributes to acceleration of ethanol metabolism (Fig. 13D).

From the results obtained above, caffeine + serine intake is conceivably effective in mitigating a physical burden at the time of alcohol drinking not only in a person of ND but also in a person of NN

### <Example 7>

### Change of blood ethanol in rat by caffeine + serine intake after ethanol administration

To male Wistar rats (Japan SLC) having a body weight of 250 g to 300 g, 2 g/kg body weight of ethanol was orally administered. One hour later, water was orally administered to a control group (n=4); 10 mg/kg body weight of caffeine (Wako Pure Chemical Industries, Ltd) to a group (n=5); 100 mg/kg body weight of serine (Wako Pure Chemical Industries, Ltd) to a group (n=5); and 10 mg/kg body weight of caffeine (Wako Pure Chemical Industries, Ltd) + 100 mg/kg body weight of serine (Wako Pure Chemical Industries, Ltd) to a group (n=5). Five hours after the ethanol intake, blood was taken from the aorta abdominalis under anesthesia with ether and the blood ethanol level was measured in the same manner as in Example 2.

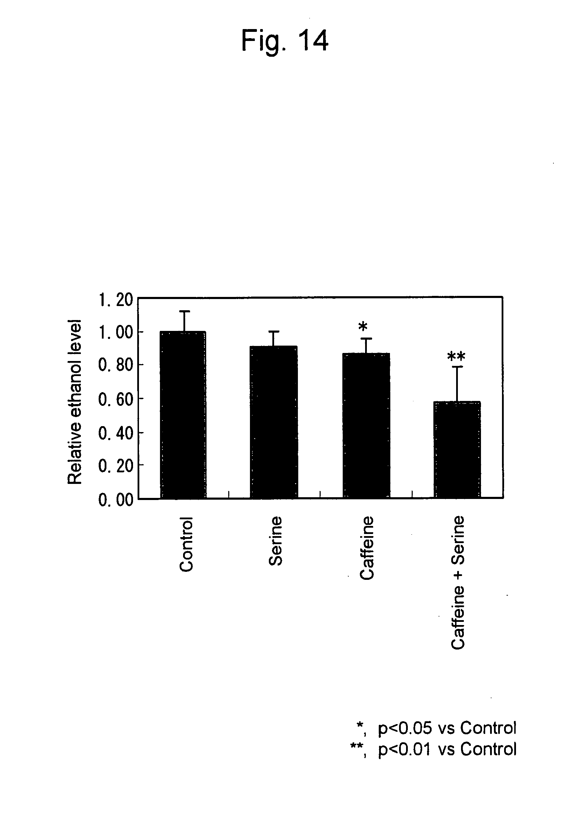

The results of relative blood ethanol concentration are shown in Fig. 14. A slight downward tendency was observed in the serine single administration group compared to the control group, but it was not significant change. In contrast, a significant reduction was observed in a caffeine administration group and a more apparent and significant reduction was observed in a caffeine + serine administration group.

These results suggest that caffeine +serine intake is effective not only before but also after ethanol intake.

### <Example 8>

### Acceleration of ethanol metabolism in human (ND) by caffeine + serine intake after ethanol intake

Change of ethanol metabolism when caffeine + serine was taken after ethanol intake was checked for a relatively light drinker (having ALDH2 genotype of ND). Six healthy adults of ND and 8 healthy adults of NN were selected as subjects for the test after obtaining informed consent. When a test was performed in the morning, they were asked to take neither meal nor water from the wake-up time to participate in the test. When a test was performed in the afternoon, the subjects were asked to take neither meal nor water after taking breakfast in the same manner as in Example 5. First, 5% ethanol solution was orally given so as to be 0.4 g/kg body weight of ethanol. Immediately before blood sampling performed 30 minutes later, 3 mg/kg body weight of caffeine (SHIRATORI Pharmaceutical Co., Ltd.) and 30 mg/kg body weight of serine (KYOWA HAKKO KOGYO Co., Ltd.) were orally administered. Blood was taken immediately before alcohol intake, 30, 60, 90, 120, and 180 minutes after the alcohol intake. The ethanol, acetaldehyde and acetate levels were measured by gas chromatography in the same manner as in Example 3. Furthermore, part of the blood was subjected to measurement of lactate level and pyruvate level in the blood. The ratio of NADH/NAD⁺ was calculated from the ratio of lactate/pyruvate.

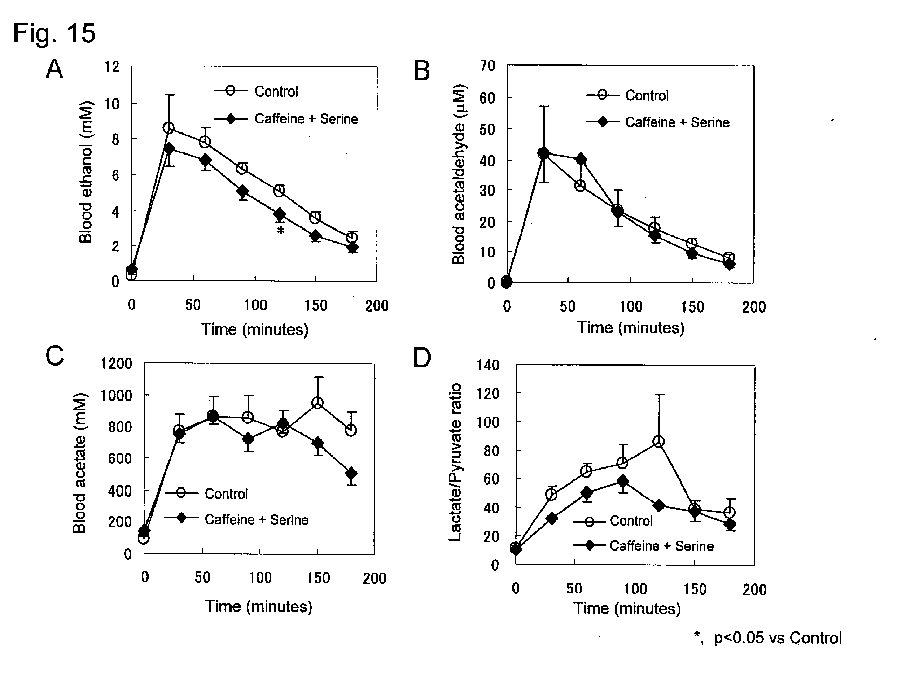

The blood ethanol level generally showed a downward tendency by caffeine + serine intake (Fig. 15A) and the acetaldehyde level showed a downward tendency on and after 120 minutes (Fig. 15B). Furthermore, a temporal increase of the blood acetate level caused by ethanol intake greatly reduced on and after 150 minutes (Fig 15C), suggesting that ethanol metabolism may be terminated earlier. Moreover, the ratio of lactate/pyruvate in blood reflecting the ratio of NADH/NAD⁺ was lower than that of the control by caffeine +serine intake. Thus, it was deduced that the conversion from NADH into NAD⁺ caused by the acceleration of the gluconeogenesis system had contributed to ethanol metabolism (Fig. 15D).

From the results obtained above, caffeine + serine intake is effective in mitigating a physical burden in a human ofND also when it is taken after alcohol drinking.

### <Example 9>

### Acceleration of ethanol metabolism in human (NN) by caffeine + serine intake after ethanol intake

Change of ethanol metabolism when caffeine + serine was taken after ethanol intake was checked for a relative hard drinker (having ALDH2 genotype of NN), in the same manner as in Example 8.

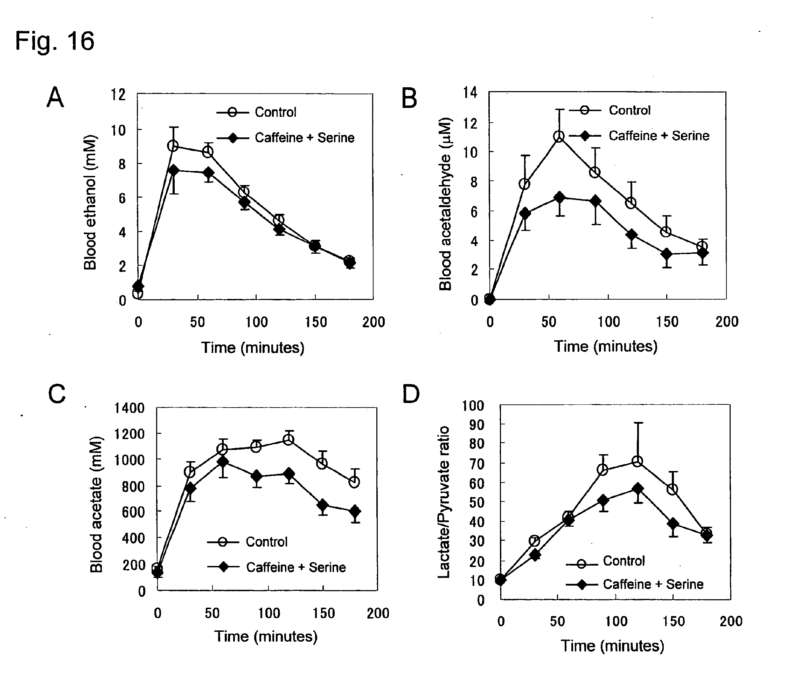

The blood ethanol level went generally decreased slightly due to caffeine + serine intake (Fig. 16A) and the acetaldehyde concentration showed a more apparent reduction (Fig. 16B). Furthermore, the blood acetate level was greatly reduced by the ethanol on and after 90 minutes (Fig 16C), suggesting that ethanol metabolism is terminated earlier. Moreover, the ratio of lactate/pyruvate in blood reflecting the ratio of NADH/NAD⁺ was lower than in the control by caffeine +serine intake. It was therefore deduced that the conversion from NADH into NAD⁺ caused by the acceleration of the gluconeogenesis system contributed to ethanol metabolism (Fig. 16D).

From the results obtained above, intake of caffeine + serine is effective in mitigating a physical burden when it is taken after alcohol drinking not only in a human ofND but also in a human ofNN.

### <Example 10>

### Effect of caffeine +serine on hangover when taken before and after alcohol drinking

Twenty healthy adults were employed as subjects after obtaining informed consent. Questionnaire survey was performed on hangover symptoms on the next morning when the caffeine + serine were taken before and after alcohol drinking. For the test, bags of granules containing 100 mg of caffeine (manufactured by SHIRATORI Pharmaceutical Co., Ltd.) and 1500 mg of serine (manufactured by KYOWA HAKKO KOGYO Co., Ltd.) per bag were prepared. The intake dose was limited to 1 to 2 bags from a safety point of view. The test period was set at about 1.5 months. In a daily occasion of alcohol drinking, subjects were asked to try 1) not to take the sample, 2) to take the sample before alcohol drinking, and 3) to take the sample after alcohol drinking, and then were asked to answer about physical conditions on the next morning in the form of questionnaire. In the questionnaire survey, the subjects were asked of 9-ranks evaluation on each item of hangover symptoms including awakening, headache, loss of appetite, nausea, upset stomach, thirsty, weakness, shivering, diarrhea, and dizziness (Wise J et al., Arch. Intern. Med. Vol164, 1334-1340 (2004)).

Fig. 17 shows the results when the sample was taken before alcohol drinking. The hangover score obtained by summing up the scores of individual items suggested that 3/4 entire hangover symptoms were improved (Fig. 17A). As to symptoms of hangover such as awakening (Fig. 17B), headache (Fig. 17C) and thirsty (Fig. 17F), about a half of the subjects answered "improved". As to loss of appetite (Fig. 17D) and upset stomach (Fig. 17E), about 1/4 to 1/3 subjects answered "improved".

As shown in Fig. 18A, also when the sample was taken after alcohol drinking, the improvement of the hangover score was confirmed in about half of all subjects. As to hangover symptoms such as awakening (Fig. 18B) and headache (Fig. 17C), about a half of the subjects answered "improved". As to loss of appetite (Fig. 18D), about 2/5 subjects and thirsty (Fig. 17E), about 1/4 subjects answered "improved".

From the results above, it is conceivable that caffeine +serine intake before and after alcohol drinking is effective in mitigating various hangover symptoms on the next morning.

Note that part of subjects were asked to take 50 mg caffeine +1000 mg serine or 50 mg caffeine + 500 mg serine. In this case, the hangover symptoms were observed to improve.

### <Example 11>

### Recovery of mice from hypoglycemia at the time of fatigue by caffeine + serine administration

A swimming experiment of male Balb/c mice (Japan SLC) was performed under flowing water by use of a flowing-water vessel (developed by Matsumoto in Kyoto University). The mice (4 weeks old) were allowed to swim once in two different days at a flow rate of about 4.5 L/min (about 16 cm/sec) for 15 to 20 minutes to allow the mice to get use to the swimming environment. On the next week, these mice (5 weeks old) were fasted for 3 hours. After that, blood was taken from the tail and blood glucose level was measured by GLUTESTACE (Sanwa Kagaku Kenkyusho Co., Ltd.). Immediately after this, the mice were allowed to swim at a flow rate of about 5.5 L/min (about 19 cm/sec), in an intermittent manner. Intermittent swim was performed repeating a cycle consisting of 10-minute swim and 10-minute rest, twice, and then repeating a cycle consisting of 5-minute swim and 10-minute rest until the mice became too exhausted to swim. After the mice too exhausted to swim were allowed to leave alone for 15 minutes, the blood glucose level was checked. After confirming reduced blood glucose level, water was orally administered to a control group (n=9), 80 mg/kg body weight of caffeine (Wako Pure Chemical Industries, Ltd) to a group of n=6, 200 mg/kg body weight of serine (Wako Pure Chemical Industries, Ltd) to a group of n=4, and 80 mg/kg body weight of caffeine + 200 mg/kg body weight of serine to a group of n=5. A blood glucose level was measured with time.

As shown in Fig. 19, a blood glucose level was quickly recovered from hypoglycemia at the time of fatigue by caffeine + serine administration, compared to the control. Improvement of the blood glucose level at 15, 45, 60, and 90 minutes after the administration had a significance (p<0.05) compared to the control. When caffeine and serine were singly administered, the mice were recovered from hypoglycemia; however, degree of recovery in the case of caffeine + serine intake was more excellent.

### <Example 12>

### The amount of caffeine and serine contained in various types of commercially available beverages

The caffeine and serine amounts of commercially available beverages, general drinks known to contain caffeine, including green tea, oolong tea, black tea, coffee, cola and a drinking preparation, were measured by an HPLC method. As shown in the Tables, the caffeine concentrations of green tea, oolong tea and black tea fell within the range of 10-21 mg/100 ml (Table 1) and the serine concentrations of these were less than 0.5 mg/100 ml, extremely low (Table 2). The caffeine concentrations of coffee and a drinking preparation were relatively high within 50-100 mg/100 ml; however, the serine concentrations fells within a detection limit or less, which were presumed to be extremely low. The caffeine concentration of cola was 11 mg/100 ml, which is as the same as those of teas and no serine was detected.

From the result, it was suggested that a caffeine containing beverage containing 100 mg/100 ml or more of serine is novel.

**[Table 1]**

| | Samples (n) | Caffeine (mg/100 ml) |
|---|---|---|
| Green tea | 12 | 12-21 |
| Oolong tea | 4 | 15-21 |
| Black tea | 3 | 10-21 |
| Coffee | 2 | 58-100 |
| Cola | 1 | 11 |
| Drinking preparation | 1 | 55 |

**[Table 2]**

| | Samples (n) | Serine (mg/100 ml) |
|---|---|---|
| Green tea | 4 | 0.2-0.5 |
| Oolong tea | 2 | <0.3 |
| Black tea | 3 | 0.2-0.5 |
| Coffee | 2 | Not detectable |
| Cola | 1 | Not detectable |
| Drinking preparation | 1 | Not detectable |

### <Example 13>

### Preparation of a beverage supplemented with serine

Beverages were prepared by adding serine (KYOWA HAKKO KOGYO Co., Ltd.) in amounts of 100, 500, 1000, 1500 and 2000 mg to commercially available non-sugar coffees of 100 ml. Serine is known as a highly soluble amino acid and dissolved in an amount of 30 g or more at room temperature, and in an amount of 20 g even at 0°C in 100 g of water. Therefore, serine of the aforementioned amount was easily dissolved in non-sugar coffee without any appearance problem such as precipitation and turbidity. In addition, serine is known as a sweet amino acid. When serine was added within the aforementioned range, coffee was sweetened to the same extent as coffee containing a small amount of sugar. Taste of the coffee became as mild as sweetened coffee. Although evaluation of the taste varies between individuals, most persons sensed a sweet taste in an amount of 500 to 1000 mg (of serine).

All publications, patents and patent applications cited in the specification are incorporated herein by reference in its entirety.

### Free text of Sequence Laiting

SEQ ID NO: 1:Description of artificial sequence: sense primer of SDS;
SEQ ID NO: 2:Description of artificial sequence: antisense primer of SDS
SEQ ID NO: 3:Description of artificial sequence: sense primer of TDO
SEQ ID NO: 4:Description of artificial sequence: antisense primer of TDO;
SEQ ID NO: 5 description of artificial sequence: sense primer of ALT1;
SEQ ID NO: 6:Description of artificial sequence: antisense primer of ALT1;
SEQ ID NO: 7:Description of artificial sequence: sense primer of P0;
SEQ ID NO: 8:Description of artificial sequence: antisense primer of P0.

## Claims

1. A composition for cooperatively accelerating alcohol metabolism and gluconeogenesis *in vivo,* **characterized by** comprising an effective amount of caffeine and an effective amount of an amino acid(s) and/or a peptide(s) selected from the group consisting of serine, tryptophan, serine rich peptides, tryptophan rich peptides, serine/tryptophan rich peptides and mixtures thereof, as active ingredients.

2. The composition according to claim 1, for use in mitigating a physical burden arising due to alcohol intake and/or accelerating alcohol metabolism.

3. The composition according to claim 1, comprising caffeine and serine as active ingredients.

4. The composition according to claim 1, comprising caffeine and tryptophan as active ingredients.

5. The composition according to claim 1, comprising caffeine and a mixture of serine and tryptophan as active ingredients.

6. The composition according to claim 1, wherein the serine rich peptide contains serine residues in an amount of 50 to 100% of the amino acids constituting the peptide.

7. The composition according to claim 6, wherein the serine-rich peptide is seryl-serine.

8. The composition according to claim 1, wherein the tryptophan rich peptide contains tryptophan residues in an amount of 50 to 100% of the amino acids constituting the peptide.

9. The composition according to claim 8, wherein the tryptophan-rich peptide is tryptophanyl-tryptophan.

10. The composition according to claim 1, wherein the total number of serine residues and tryptophan residues of the serine/tryptophan rich peptide accounts for 50% to 100% of the amino acids constituting the peptide.

11. The composition according to claim 10, wherein the serine/tryptophan-rich peptide is seryl-tryptophan or tryptophanyl-serine.

12. The composition according to claim 1, wherein the content of caffeine falls within an acceptable intake range of a human per composition.

13. The composition according to claim 12, wherein the content of caffeine is 500 mg or less per composition.

14. The composition according to claim 13, wherein the content of caffeine is 50 mg to 500 mg per composition.

15. The composition according to claim 1, wherein the content of the amino acid is 100 mg or more per composition.

16. The composition according to claim 15, wherein the content of the amino acid is 100 mg to 20 g per composition.

17. The composition according to claim 1, wherein the content of the peptide is 100 mg or more per composition.

18. The composition according to claim 1, wherein the content of serine is 500 mg or more per composition in terms of free serine.

19. The composition according to claim 1, wherein the content of serine is 1 g or more per composition in terms of free serine.

20. The composition according to claim 1, being a beverage or food or a medicament.

21. A composition comprising 500 mg or less of caffeine and 100 mg or more in total of a combination of at least 2 components selected from the group consisting of serine or serine rich peptides, tryptophan or tryptophan rich peptides, and serine/tryptophan rich peptides.

22. The composition according to claim 21, wherein the content of serine in the combination is 100 mg or more per composition in terms of free serine.

23. The composition according to claim 21, wherein the content of serine in the combination is 500 mg or more per composition in terms of free serine:

24. The composition according to claim 21, wherein the content of serine in the combination is 1 g or more per composition in terms of free serine.

25. The composition according to claim 21, being a beverage or food or medicament.

26. A composition containing 500 mg or less of caffeine and 100 mg or more of a serine/tryptophan rich peptide.

27. The composition according to claim 26, being a beverage or food or medicament.

28. A composition for relieving fatigue, **characterized by** comprising an effective amount of caffeine and an effective amount of an amino acid(s) and/or a peptide(s) selected from the group consisting of serine, tryptophan, serine rich peptides, tryptophan rich peptides, serine/tryptophan rich peptides and mixtures thereof, as active ingredients.

29. The composition according to claim 28, for use in mitigating a physical burden arising due to alcohol intake.

30. The composition according to claim 28, for use in relieving fatigue arising due to hypoglycemia.

31. The composition according to claim 28, comprising caffeine and serine as active ingredients.

32. The composition according to claim 28, being a beverage or food or medicament.

33. A composition for accelerating alcohol metabolism, **characterized by** comprising an effective amount of caffeine and an effective amount of an amino acid(s) and/or a peptide(s) selected from the group consisting of serine, tryptophan, serine rich peptides, tryptophan rich peptides, serine/tryptophan rich peptides and mixtures thereof, as active ingredients.

34. The composition according to claim 33, comprising caffeine, and serine, tryptophan or a mixture thereof as active ingredients.

35. The composition according to claim 33, wherein the content of serine is 500 mg or more per composition in terms of free serine.

36. The composition according to claim 33, wherein the content of serine is 1 g or more per composition in terms of free serine.

37. The composition according to claim 33, being a beverage or food or medicament.

38. A composition set comprising a first composition containing 500 mg or less of caffeine and a second composition containing 100 mg or more of serine, tryptophan, or a mixture thereof in combination as discrete packages.

39. The composition according to claim 38, wherein the content of serine in the second composition is 500 mg or more.

40. The composition according to claim 38, wherein the content of serine in the second composition is 1 g or more.

41. The composition according to claim 38, being a beverage or food or medicament.

42. A caffeine containing beverage, **characterized by** comprising 500 mg or more of serine per beverage.

43. The beverage according to claim 42, selected from the group consisting of green tea, black tea, coffee, cola and a drinkable preparation.

44. The beverage according to claim 42, wherein the content of caffeine is 10 mg to 500 mg, both inclusive.

45. The beverage according to claim 42, wherein the content of caffeine is 50 mg to 500 mg, both inclusive.

46. The beverage according to claim 42, wherein the content of caffeine is 100 mg to 500 mg, both inclusive.

47. The beverage according to claim 42, wherein the content of caffeine is 150 mg to 500 mg, both inclusive.

48. A composition to be incorporated in a caffeine containing beverage for use in accelerating alcohol metabolism or relieving fatigue in mixing, **characterized by** supplementally adding 100 mg or more of serine per dose.

49. The composition according to claim 48, wherein the content of serine is 500 mg or more.

50. The composition according to claim 48, wherein the composition is selected from the group consisting of sugar, milk and lemon juice.
